# EUROPÄISCHE PATENTANMELDUNG

(11) **EP 4 112 609 A1**
(43) Veröffentlichungstag der Anmeldung: **04.01.2023**
(21) Anmeldenummer: 21182701.9
(22) Anmeldetag: 30.06.2021
(51) Int. Cl.: C07D 301/03, C07D 301/10, B01J 23/66

(54) **VERFAHREN ZUR HERSTELLUNG VON ALKYLENOXIDEN**

(71) Anmelder: Covestro Deutschland AG, 51373 Leverkusen (DE)
(72) Erfinder: Leven, Matthias, 51069 Köln (DE); Heinz, Norah, 50969 Köln (DE); Liman, Ulrich, 40764 Langenfeld (DE); Langanke, Jens, 53894 Mechernich (DE); Heinemann, Torsten, 42799 Leichlingen (DE)
(74) Vertreter: Levpat

(57) **Zusammenfassung**

Gegenstand der Erfindung ist ein Verfahren zur Herstellung eines Alkylenoxids durch Umsetzung eines Alkens mit einem Arenoxid, Pyridin-N-oxid und/oder Pyrimidin-N-oxid bevorzugt mit einem Arenoxid und/oder Pyridin-N-oxid bevorzugt in Gegenwart eines Katalysators in einem ersten Reaktor, wobei der Katalysator ein Metall und/oder ein Metallsalz umfasst, wobei das Metall Kupfer, Silber und/oder Gold ist, wobei das Metallsalz Chrom, Eisen, Cobalt und/oder Kupfer, Kation(en) umfasst, und wobei die Umsetzung in Abwesenheit von Sauerstoff oder einem Sauerstoff-haltigen Gasgemisch erfolgt.

## Beschreibung

Gegenstand der Erfindung ist ein Verfahren zur Herstellung eines Alkylenoxids durch Umsetzung eines Alkens mit einem Arenoxid, Pyridin-N-oxid und/oder Pyrimidin-N-oxid bevorzugt mit einem Arenoxid und/oder Pyridin-N-oxid bevorzugt in Gegenwart eines Katalysators in einem ersten Reaktor, wobei der Katalysator ein Metall und/oder ein Metallsalz umfasst, wobei das Metall Kupfer, Silber und/oder Gold ist, wobei das Metallsalz Chrom, Eisen, Cobalt und/oder Kupfer, Kation(en) umfasst, und wobei die Umsetzung in Abwesenheit von Sauerstoff oder einem Sauerstoff-haltigen Gasgemisch erfolgt.

Im Stand der Technik sind zahlreiche Verfahren zur Herstellung von Alkyenoxiden, speziell Propylenoxid vorbeschrieben, wobei industriell vor allem das Chlorhydrin-Verfahren von Bedeutung ist, bei dem Propen mit unterchloriger Säure oder Chlor und Wasser einem Isomerengemisch aus 1-Chlor-2-Propanol und 2-Chlor-1-Propanol umgesetzt wird, welches im Anschluss mit Kalkmilch zu Propylenoxid und Calciumchlorid umgesetzt wird. Allerdings resultiert infolge der Bildung von CaCl₂-Salzfracht eine hohe Abwasserbelastung bzw. einen zusätzlichen Recylierungsschritt und die Einbindung in einen Chlor-Alkali-Betrieb. Diese Technologie wird als nachteilig bezüglich des Kohlenstofffußabdrucks (engl. carbon footprint) und der Treibhausgasemissionen bewertet (Reduction of GHG Emissions in Propylene Oxide Production, Approved VCS Methodology VM0023 Version 1.0, 9 September 2013 Sectoral Scope 5, South Pole Carbon Asset Management Ltd.).

Daneben ist die Herstellung von Proplyenoxid mittels Koppelprodukt-basierter Verfahren (z.B. Oxiranverfahren) von Relevanz, bei dem Ethylbenzol bzw. Iso-Butan in einer 1. Stufe in Gegenwart von Sauerstoff zu den jeweiligen Hydroperoxiden umgesetzt werden, welche im Anschluss mit Propen zu Propylenoxid und 1-Phenylethanol bzw. tert-Butanol als weitere Koppelprodukte umgesetzt werden kann. Diese Koppelprodukte können in der Folge zu Styrol und Iso-Buten bzw. Isobutan weiter umgesetzt werden. Allerdings wird neben dem Propylenoxid ebenfalls ein Koppelprodukt gebildet, welches zusätzlich abgetrennt und in weiteren Anlagen weiter verarbeitet werden muss. Die Koppelprodukt-basierten Verfahren setzen immer einen Absatzmarkt für das Koppelprodukt voraus und machen die Ökonomie solcher Verfahren komplex und anfällig für adverse Effekte. Somit liegen neben den technischen anspruchsvollen Verfahren auch noch marktwirtschaftliche Risiken auf der Hand, so dass ein techno-ökonomische Betrachtung dieser Verfahren ein wenig vorteilhaftes Gesamtbild zeichnet. Zumal die Nachfrage- bzw. Absatzsituation beider Produkte, PO und das Koppelprodukt, i.d.R. regional unterschiedlich sind. D.h. es ist eine Fernlogistik notwendig, die direkte Kosten und weitere Nachteile, wie CO2/GHG-Emissionen, mit sich bringt. Das industrielle MTBE-Verfahren benötigt eine Einbindung in einen Raffineriebetrieb, um eine maximalen ökonomischen Beitrag zu erwirtschaften.

Auch das sogenannte HPPO-Verfahren hat großtechnische Relevanz, bei dem Propylen mit Wasserstoffperoxid zu Propylenoxid und Wasser umgesetzt wird. Vorteilhaft gegenüber den vorgenannten großtechnischen Herstellungsverfahren ist, dass kein Koppelprodukt oder Salzlasten im Produkt resultieren. Allerdings muss Wasserstoffperoxid in einem vorgeschalteten, technisch komplexen katalytischen Prozess hergestellt werden. Das Verfahren gilt als technisch anspruchsvoll und wird i.d.R. nur an Verbundstandorten mit anderen H2O2-Verbrauchern betrieben.

Die Direktoxidation von Propen zu Propylenoxid gilt weiterhin als technisch nicht ausgereift und nach jetzigem Stand der Technik gibt es keinen Direktoxidationsprozess, der industriell ökonomisch durchführbar ist. Insbesondere die Reaktionsführung und besonders die Temperaturkontrolle in Gasphasen- und Salzschmelzenprozessen sind eine ungelöste Herausforderung, wobei jedoch eine hoher Propen-Umsatz in Verbindung mit einer hohen PO-Selektivität für ein effizientes technisches Verfahren maßgeblich ist. Bei der Direktoxidation treten eine Reihe von Neben- und Folgeprodukten, wie z.B. Methanol, Acetaldehyd, Kohlendioxid, Ethylen and Formaldehyd, in substantiellen Mengen auf (vgl. D. Kahlich et. al., Dow Deutschland in Ullmann's Encyclopedia of Industrial Chemistry, Kapitel "Propylene Oxide", Wiley VCH, 2012). Z.B. können als aktuelle Dokumente des Stands der Technik für die aerobe Oxidation von Alkene wie Propene US2018208569A1 und US2020346193A1 herangezogen werden. Lediglich 54,5 % bzw. 60 % Epoxid-Selektivität werden bei 8.6 % bzw. 5 % Umsatz erzielt.

Arenoxide sind zentrale Schlüsselverbindungen des oxidativen Stoffwechsels von aromatischen Verbindungen. Sie können durch enzymatische Syntheseverfahren in hohen Ausbeuten dargestellt werden. Wissenschaftliche Publikationen die diese Prozesse zu Zwecken der Synthese ausführlich ausführen sind beispielweise "Enzymatic and chemoenzymatic synthesis of arene transdihydrodiols" von Journal of Molecular Catalysis B: Enzymatic, Volumes 19-20, 2, 2002, 31-42 oder "Chemical Equivalent of Arene Monooxygenases: Dearomative Synthesis of Arene Oxides and Oxepines" von Zohaib Siddiqi, William C. Wertjes, and David Sarlah, J. Am. Chem. Soc. 2020, 142, 22, 10125-10131. Aber auch andere Syntheseverfahren wie direkte Epoxidierung, Darstellung aus trans-1,2-Glykolen, Darstellung aus *cis*-1,2-Glykole, Darstellung aus Bromohydrinen, Darstellung aus vicinalen Dihalogen-Verbindungen von epoxidierten Proaromaten, Ringschluss von *seco* Derivaten, electrochemische Oxidation, Umsetzung von Annulenen, Umsetzung von Arenphotooxiden und/oder -peroxiden, Umsetzung von Ozonid-Verbindungen, Darstellung aus Sauerstoffheterocyclen sowie durch intramolekulare Sauerstoffübertragsreaktionen (engl.: intramolecular oxygen trapping) sind geeignete Herstellungsverfahren (vgl. z.B. G. S. Shirwaiker et al., Advances in Heterocyclic Chemistry, Vol. 37, 1984, 67-165).

Arenoxide werden durch die Oxidation von aromatischen und polycyclischen aromatischen Verbindungen gewonnen. Typischerweise werden dabei Hypochlorite als Oxidationsmittel in Kombination mit Phasentransferkatalysatoren eingesetzt (vgl. z.B. JP61109784). Die Reaktionen werden entweder in Substanz oder in Lösungsmittel durchgeführt, dabei haben sich insbesondere chlorierte und nitrierte Kohlenwasserstoffe bewährt. Eine pH-Kontrolle kann vorteilhaft bei der Synthese sein.

Arenoxide verschiedener halogenierter Benzole sind auch kommerziell erhältlich.

Die selektive Oxidation von ternären Stickstoffverbindungen ist sehr gut beschrieben. Z.B. sind Verfahren unter Verwendung von Wasserstoffperoxid und dem Einsatz von Methyltrioxorhenium(VII) (J. Org. Chem., 1995, 60 , 1326), Magnesium-Porphyrinen (Synthesis, 1997, 1387), Flavinen (1980, 102 , 6498),TS-1 und anderen Zeolithen (Catal. Lett., 2001, 72 , 233), Molsieb (Chem. Commun., 2000, 1577), Polyoxometallaten (J. Mol. Catalysis A: Chem. 252, 219-225, 2006; Green Chem., 2011, 13, 1486-1489) oder Wolfram-substituierten Mg-Al Schichthydroxiden (Chem. Commun., 2001, 1736) als Katalysatoren beschrieben. Zudem sind dem Fachmann andere Syntheseverfahren unter Verwendung von unterschiedlichen anorganischen und organischen Oxidationsmittel wie z.B. organische Persäuren, Dioxirane, Peroxomonoschwefelsäure (*Caro* Säure), Peroxomonophosphorsäure, usw. bekannt (vgl. u.a. Michael B. Smith, March's Advanced Organic Chemistry, 7th Edition, Wiley, 2012).

N-Oxide von aromatischen, N-heterocyclischenVerbindungen lassen sich durch die Oxidation mit Hilfe von Sauerstoff und Ruthenium(III)chlorid als Katalysator gewinnen (S. L. Jain et al. Chem. Commun. 2002, 1040-1041). Bei 20 °C und 1 atm O₂ lassen sich N-Oxide von Pyridin, 2-, 3- und 4-Picoline, substituierten Pyridinen, Chinolin und Isochinolin in Ausbeuten von bis zu 95 % gewinnen. Der Einsatz von bestimmten nicht-protischen Lösungsmitteln und/oder zusätzlichen P-Liganden kann die Kinetik des Verfahrens positiv beeinflussen.

CN108623519 beschreibt die Oxidation von Pyridin mit Sauerstoff in Isopropanol unter Anwesenheit eines Titan-haltigen Zeolith -Katalysators. Es handelt sich um einen technisch einfachen Prozess sowie hohe Umsätze und hohe Ausbeuten werden angegeben.

Die Nutzung von N-Oxiden als Oxidationsmittel ist bekannt (vgl. z.B. Org. Synth. Coll. Vol., 1988, 6, 342; Chem. Rev., 1980, 80, 187; Org. Proc. Res. Dev., 1997, 1, 425; Synthesis, 1994, 639; Chem. Ber., 1961, 94, 1360; Bull. Chem. Soc. Jpn., 1986, 59, 3287; Tetrahedron Lett., 1990, 31, 4825; J. Chem. Soc., Chem. Commun., 1987, 1625). Dabei zeichnen sich N-Oxide als effiziente Überträger von atomarem Sauerstoff aus, wobei die ursprüngliche Stickstoffverbindung zurückgewonnen wird. Diese kann dann einfach recycelt werden oder *in situ* direkt in das dementsprechende N-Oxid oxidativ zurückgeführt werden. Beim Einsatz von N-oxidischen Oxidantien findet eine Überoxidation und die Bildung von Neben- und/oder Folgeprodukten i.d.R. nicht statt, insbesondere wenn selektive Katalysatorsysteme eingesetzt werden.

Die selektive katalytische Umsetzung von ungesättigten Verbindungen zu Epoxiden unter Verwendung von N-Oxiden ist in der Literatur gut beschrieben (RSC Adv., 2016, 6, 88189-88215). Bisher wurde die Epoxidierung von nicht-aromatischen, linearen α-Olefinen mit N-Oxiden nur unter Einsatz aufwendiger Ruthenium-basierte Porpyhyrin-Katalysatoren und sehr langen Reaktionszeiten bewerkstelligt: Z.B. wurde bei der Epoxidierung von 1-Octen unter Einsatz eines Ruthenium-Porphyrin Katalysators (Summenformel C₈₅H₇₆ON₄Ru) und 2,6- Dichloropyridin N-oxid in Benzol als Lösungsmittel bei 125 °C in einem Schlenkrohr nach 48 Stunden ein Umsatz von lediglich 6 % und eine Epoxidausbeute von 5% erzielt (J. Chem. Soc., Perkin Trans. 1, 1997). In einem weiteren Beispiel wurde bei der Epoxidierung von 1-Octen unter Einsatz eines MCM-41 geträgerten Ruthenium-Porphyrin Katalysators und 2,6-Dichloropyridin N-oxid in Dichlormethan als Lösungsmittel bei 40 °C nach 24 Stunden ein Umsatz von 80 % und eine Epoxidausbeute von 74% erzielt (J. Org. Chem., 1998, 63, 7364-7369). In einem weiteren Beispiel wurde bei der Epoxidierung von 1-Octen unter Einsatz eines PEGylierten Ruthenium-Porphyrin Katalysators und 2,6-Dichloropyridin N-oxid in Dichlormethan als Lösungsmittel bei 50 °C nach 24 Stunden ein Umsatz von 82 % und eine Epoxidausbeute von 81% erzielt (Chem.-Eur. J., 2006, 12, 3020-3031). Eine theoretische Arbeit (RSC Adv., 2016, 6, 88189-88215) schlägt den Einsatz von Ru(meso-tetrakis(2,6-dichlorophenyl)porphyrin) als Katalysator in Kombination mit Dimethylpyridin N-oxid zur Epoxidierung von Propen zu Propylenoxid vor ohne Reaktionsbedingungen anzugeben. Insgesamt zeigen die niedrigen Reaktionstemperaturen in Kombination mit den langen Reaktionszeiten, dass diese Ru-basierten Katalysatoren nur einen geringen Mehrwert für den technischen Einsatz in der Epoxidierung von linearen, terminalen Alkenen haben. Es lassen sich nur brauchbare Selektivitäten erzielen, wenn sehr niedrige Reaktionstemperaturen gewählt werden. Zudem kommen Lösungsmittel zum Einsatz, deren Einsatz im großtechnischen Umfang nicht zu bevorzugen sind. Die im Stand der Technik eingesetzten Katalysatoren enthalten Ru in den Oxidationsstufen (+II) und (+IV).

Daher war es Aufgabe der vorliegenden Erfindung ein Katalysatorsystems für die direkte Herstellung von Alkylenoxiden (Epoxiden) bevorzugt von Propylenoxid bereitzustellen, welches in der oxidativen Umsetzung von Alkenen in Abwesenheit von Sauerstoff oder einem sauerstoff-haltigen Gasgemisch ein im Vergleich zu aus dem Stand der Technik bekannten Systemen mit verbessertem Epoxid- Umsatz sowie eine verbesserte Produktselektivität durch Reduktion der Bildung unerwünschter Nebenprodukte; wie allylische Verbindungen und deren Folgeprodukte, und die Vermeidung der Bildung von nicht rezyclierbaren Koppelprodukten aufweist. Diese verbesserte Katalysatoraktivität sowie Selektivität sollte sich durch eine Aktivierungsenergie E_{A} von bis zu 24.0 kcal/mol bevorzugt von 10.0 kcal/mol bis21.0 kcal/mol für den Sauerstoffübertrag auf das Alken bevorzugt das Propen widerspiegeln, wobei Neben- oder Folgereaktionen höhere Aktivierungsenergien aufweisen als die bevorzugte Alkoxylierungsreaktion. Höhere Aktivierungsenergien von mehr als 24.0 kcal/mol würden höhere Reaktionstemperaturen erfordern, welche die bereits diskutierten, aber auch weitere, unerwünschten Nebenreaktionen begünstigen. Zudem sollten die Aktivierungsenergien von Neben- oder Folgereaktionen größer gleich 10.0 kcal/mol sein damit eine Selektivitätskontrolle über die Reaktionstemperatur größer 20 °C möglich ist.

Überraschend wurde gefunden, dass die erfindungsgemäße Aufgabe gelöst wird durch ein Verfahren zur Herstellung eines Alkylenoxids durch Umsetzung eines Alkens mit einem Arenoxid, Pyridin-N-oxid und/oder Pyrimidin-N-oxid bevorzugt mit einem Arenoxid und/oder Pyridin-N-oxid in Gegenwart eines Katalysators (A) in einem ersten Reaktor, wobei der Katalysator (A) ein Metall (A-1) und/oder ein Metallsalz (A-2) umfasst, wobei das Metall (A-1) Kupfer, Silber und/oder Gold ist, wobei das Metallsalz (A-2) Chrom (Cr), Eisen (Fe), Cobalt (Co) und/oder Kupfer (Cu) -Kation(en) umfasst, und wobei die Umsetzung in Abwesenheit von Sauerstoff oder einem Sauerstoff-haltigen Gasgemisch erfolgt.

Die nachfolgenden Ausführungsformen können beliebig kombiniert werden, insofern sich aus dem technischen Kontext und dem allgemeinen Fachwissen nicht das Gegenteil ergibt.

Das erfindungsgemäße Alkylenoxid (Epoxid) kann ein Alkylenoxid mit 2-45 Kohlenstoffatomen sein. In einer Ausführungsform des erfindungsgemäßen Verfahrens wird das Alkylenoxid ausgewählt aus mindestens einer Verbindung der Gruppe bestehend aus Ethylenoxid, Propylenoxid, 1,2-Butylenoxid, 2,3-Butylenoxid, 2-Methyl-1,2-propylenoxid (Isobutenoxid), 1,2-Pentylenoxid, 2,3-Pentylenoxid, 2-Methyl-1,2-butylenoxid, 3-Methyl-1,2-butylenoxid, Alkylenoxide von C6-C22 α-Olefinen, wie 1,2-Hexylenoxid, 2,3-Hexylenoxid, 3,4-Hexylenoxid, 2-Methyl-1,2-pentylenoxid, 4-Methyl-1,2-pentylenoxid, 2-Ethyl-1,2-butylenoxid, 1,2-Heptylenoxid, 1,2-Octylenoxid, 1,2-Nonylenoxid, 1,2-Decylenoxid, 1,2-Undecylenoxid, 1,2-Dodecylenoxid, 4-Methyl-1,2-pentylenoxid, Cyclopentylenoxid, Cyclohexylenoxid, Cycloheptylenoxid, Cyclooctylenoxid, Styroloxid, Methylstyroloxid, Pinenoxid, Allylglycedylether, Vinylcyclohexylenoxid, Cyclooctadienmonoepoxid, Cyclododecatrienmono-epoxid, Butadienmonoepoxid, Isoprenmonoepoxid, Limonenoxid, 1,4-Divinylbenzolmonoepoxid, 1,3-Divinylbenzolmonoepoxid, Glycidylacrylat und Glycidylmethacrylat ein- oder mehrfach epoxidierte Fette als Mono-, Di- und Triglyceride, epoxidierte Fettsäuren, C1-C24-Ester epoxidierter Fettsäuren, Epichlorhydrin, Glycidol, und Derivate des Glycidols wie beispielsweise Glycidylether von C1-C22 Alkanolen, Glycidylester von C1-C22 Alkancarbonsäuren. Beispiele für Derivate des Glycidols sind Phenylglycidylether, Kresylglycidylether, Methylglycidylether, Ethylglycidylether und 2-Ethylhexylglycidy lether.

In einer bevorzugten Ausführungsform des Verfahrens ist das Alkylenoxid Ethylenoxid, Propylenoxid, 1,2-Butylenoxid, 1,2-Pentylenoxid, 1,2-Hexylenoxid, 1,2-Heptylenoxid und/oder 1,2-Octylenoxid. In einer besonders bevorzugten Ausführungsform des Verfahrens ist das Alkylenoxid Ethylenoxid und/oder Propylenoxid. In einer ganz besonders bevorzugten Ausführungsform des Verfahrens ist das Alkylenoxid Propylenoxid.

In einer Ausführungsform des erfindungsgemäßen Verfahrens ist das Alken eine oder mehrere Verbindung(en) und wird ausgewählt aus der Gruppe bestehend aus Ethen, Propen, Buten, 1-Okten, Butadien, 1,4-Butandioldiallylether, Allylchlorid, Allylalcohol, Styrol, Cyclopenten, Cyclohexen, Phenyl-allylether, Diallylether, n-Butyl-allylether, tert-Butyl-allylether, Bisphenol-A diallyl ether, Resorcindiallylether, Triphenylolmethan-triallylether, Cyclohexan-1,2-dicarbonsäure-bis-(allylester), Isocyanursäuretris-(prop-2,3-en)-ester und Gemische dieser Alkene bevorzugt Ethen, Propen und Allylchlorid und besonders bevorzugt Propen.

In einer Ausführungsform des erfindungsgemäßen Verfahrens ist das Arenoxid eine oder mehrere Verbindungen gemäß Formel (I), (II), (III) und/oder (IV): 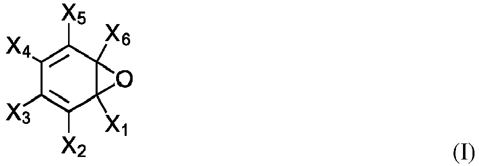 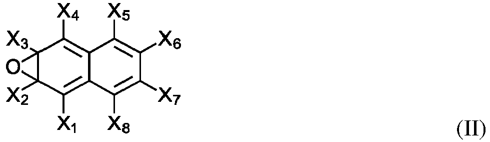 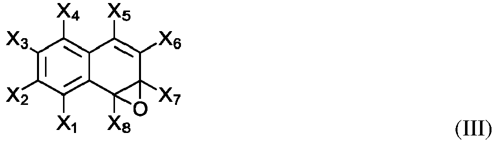 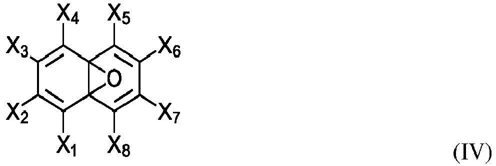 mit
X₁ bis X₈ unabhängig voneinander ausgewählt aus der Gruppe F, Cl, Br, CN, -CO-CF₃, -CO-C(CH3)₃, -CO-CH₂C(CH₃)₃, -CO-C₆H₅, -CO-OC(CH₃)₃, -CO-OCH₂C(CH₃)₃ oder H bevorzugt F, Cl besonders bevorzugt Cl.

In einer bevorzugten Ausführungsform des erfindungsgemäßen Verfahrens ist das Arenoxid eine oder mehrere Verbindungen und wird ausgewählt aus der Gruppe bestehend aus Hexafluorbenzoloxid, Hexachlorbenzoloxid, 1-Brom-2,3,4-trifluorbenzoloxid, Pentafluorbenzoloxid, 1,3,5-Trichlor-2,4,6-trifluorbenzoloxid, 1,3,5-Trfluorbenzoloxid, 1,2-Dibrom-3,5-difluorbenzoloxid, 1,2,4,5-Tetrafluorbenzoloxid, Brompentafluorbenzoloxid, 1,3,5-Trichlorbenzoloxid, 1-Brom-3,5-dichlrobenzoloxid, Orthodichlorbenzoloxid, 1,2,4,5-Tetrachlorbenzoloxid, 1,2,3-Trichlorbenzoloxid und 1,5-Dichlro-2-fluorbenzoloxid, bevorzugt Hexafluorbenzoloxid, und Hexachlorbenzoloxid.

In einer Ausführungsform des erfindungsgemäßen Verfahrens ist das Arenoxid erhältlich durch Umsetzung einer ersten aromatischen Verbindung mit Sauerstoff oder einem Sauerstoff-haltigen Gasgemisch in Gegenwart eines Katalysators (C).

In einer Ausführungsform des erfindungsgemäßen Verfahrens wird das Arenoxid hergestellt durch Umsetzung einer ersten aromatischen Verbindung mit Sauerstoff oder einem Sauerstoff-haltigen Gasgemisch in Gegenwart eines Katalysators (C).

Das sauerstoff-haltige Gasgemisch umfasst neben Sauerstoff auch Verdünnungs-, Träger- oder InertGase wie Kohlenwasserstoffe, Edelgase, CO, CO2 und/oderN2.

In einer Ausführungsform des erfindungsgemäßen Verfahrens werden dem Sauerstoff oder einem Sauerstoff-haltigen Gasgemisch weitere Additive wie Wasser, CO, N-haltige Verbindungen wie beispielsweise Hydrazin, Ammoniak (NH3), Methylamin (MeNH₂), NOx, PH₃, SO₂ und/oder SO₃ in Mengen von 10 ppm bis 500 ppm, bevorzugt von 30 ppm bis 300 ppm und besonders bevorzugt von 50 ppm bis 200 ppm zugefügt. Zudem kann CO2 in einem Anteil von 0,01 Vol-% bis 50 Vol-%, bevorzugt von 0,1 Vol-% bis 20 Vol-%, und besonders bevorzugt von 1 Vol-% bis 10 Vol-% zugesetzt werden. Außerdem können organische Halogenide wie Ethylendichlorid, Ethylchlorid, Vinylchlorid, Methylchlorid und/oder Methylenchlorid in Mengen von 10 ppm bis 500 ppm, bevorzugt von 50 ppm bis 400 ppm, und besonders bevorzugt von 100 ppm bis 300 ppm ppm zugesetzt werden.

In einer Ausführungsform des erfindungsgemäßen Verfahrens weist die erste aromatische Verbindung eine Siedetemperatur von 50 °C bis 350 °C bei 1bara auf.

In einer Ausführungsform des erfindungsgemäßen Verfahrens ist die erste aromatische Verbindung eine oder mehrere Verbindung(en) und wird ausgewählt aus der Gruppe bestehend aus Hexafluorbenzol, Hexachlorbenzol, 1-Brom-2,3,4-trifluorbenzol, Pentafluorbenzol, 1,3,5-Trichlor-2,4,6-trifluorbenzol, 1,3,5-Trifluorbenzol, 1,2-Dibrom-3,5-difluorbenzol, 1,2,4,5-Tetrafluorbenzol, Brompentafluorbenzol, 1,3,5-Trichlorbenzol, 1-Brom-3,5-dichlrobenzol, Orthodichlorbenzol, 1,2,4,5-Tetrachlorbenzol, 1,2,3-Trichlorbenzol und 1,5-Dichlro-2-fluorbenzol bevorzugt Hexafluorbenzol und Hexachlorbenzol.

In einer Ausführungsform des erfindungsgemäßen Verfahrens ist der Katalysator (C) eine oder mehrere Verbindung(en) und wird ausgewählt aus der Gruppe bestehend aus Silber, Silber geträgert auf Magnesiumsilikat und Enzym Cytochrom P450.

In einer Ausführungsform des erfindungsgemäßen Verfahrens beträgt das Stoffmengenverhältnis des Sauerstoffs zur ersten aromatischen Verbindung von 1:500 bis 1:1 bevorzugt von 1:100 bis 1:1 beträgt.

In einer Ausführungsform des erfindungsgemäßen Verfahrens wird der Katalysator (C) in einer berechneten Menge von 10 ppm bis 15%, bevorzugt von 100 ppm bis 5% und besonders bevorzugt von 100 ppm bis 2% bezogen auf die Menge der ersten aromatischen Verbindung eingesetzt.

In einer Ausführungsform des erfindungsgemäßen Verfahrens erfolgt die Herstellung des Arenoxids bei einer Temperatur von 20 °C bis 250 °C bevorzugt von 50 °C bis 220 °C und besonders bevorzugt von 100 °C bis 200 °C.

In einer Ausführungsform des erfindungsgemäßen Verfahrens erfolgt die Herstellung des Arenoxids bei einem Druck von 1 bara bis 200 bara bevorzugt von 1 bara bis 100 bara und besonders bevorzugt von 1 bara bis 60 bara.

In einer Ausführungsform des erfindungsgemäßen Verfahrens erfolgt die Herstellung des Arenoxids in einem Zeitraum von 6 min bis 48 h, bevorzugt von 6 min bis 24 h und besonders bevorzugt von 6 min bis 3 h.

In einer Ausführungsform des erfindungsgemäßen Verfahrens ist das Pyridin-N-oxids eine oder mehrere Verbindungen gemäß Formel (V): 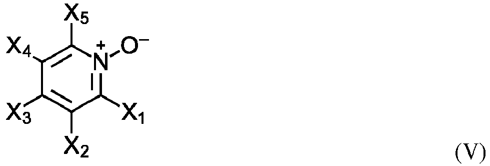 mit
X₁ bis X₅ unabhängig voneinander ausgewählt aus der Gruppe F, Cl, Br, CN, -CO-CF₃, -CO-C(CH3)₃, -CO-CH₂C(CH₃)₃, -CO-C₆H₅, -CO-OC(CH₃)₃, -CO-OCH₂C(CH₃)₃ oder H bevorzugt F, Cl besonders bevorzugt Cl.

In einer Ausführungsform des erfindungsgemäßen Verfahrens ist das Pyridin-N-oxid eine oder mehrere Verbindung(en) und wird ausgewählt aus der Gruppe bestehend aus Pentafluorpyridin-N-Oxid, 2-Brom-3,5-dichlorpyridin-N-oxid, 3-Chlorpyridin-N-oxid, 3,6-Dichlorpyridin-N-oxid, 3,5-Dichlorpyridin-N-oxid, 3-Chlor-2,5,6-trifluorpyridin-N-oxid, 3-Chlo-2,4,5,6-tetrafluorpyridin-1-N-oxid, 3-Chlo-2,4,5,6-tetrafluorpyridin-3-N-oxid, bevorzugt Pentafluorpyridin-N-Oxid.

In einer Ausführungsform des erfindungsgemäßen Verfahrens ist das Pyridin-N-oxid erhältlich durch Umsetzung einer zweiten aromatischen Verbindung mit Sauerstoff oder einem Sauerstoff-haltigen Gasgemisch in Gegenwart eines Katalysators (D).

In einer Ausführungsform des erfindungsgemäßen Verfahrens wird das Pyridin-N-oxid hergestellt durch Umsetzung einer zweiten aromatischen Verbindung mit Sauerstoff oder einem Sauerstoff-haltigen Gasgemisch in Gegenwart eines Katalysators (D).

In einer Ausführungsform des erfindungsgemäßen Verfahrens weist die zweite aromatische Verbindung eine Siedetemperatur von 50 °C bis 350 °C bei 1bara auf.

In einer Ausführungsform des erfindungsgemäßen Verfahrens ist die zweite aromatische Verbindung eine oder mehrere Verbindung(en) und wird ausgewählt aus der Gruppe bestehend aus aus Pentafluorpyridin-N-Oxid, 2-Brom-3,5-dichlorpyridin, 3-Chlorpyridin, 3,6-Dichlorpyridin, 3,5-Dichlorpyridin, 3-Chlor-2,5,6-trifluorpyridin, 3-Chlo-2,4,5,6-tetrafluorpyridin, 3-Chlo-2,4,5,6-tetrafluorpyridin, bevorzugt Pentafluorpyridin.

In einer Ausführungsform des erfindungsgemäßen Verfahrens ist der Katalysator (D) eine oder mehrere Verbindung(en) und wird ausgewählt wird aus der Gruppe bestehend aus Rutheniumtrichlorid, Titanhaltige Zeolithe und Silber.

In einer Ausführungsform des erfindungsgemäßen Verfahrens wird der Katalysator (D) auf einem Katalysatorträger aufgebracht, und der Katalysatorträger ist eine oder mehrere Verbindungen und wird ausgewählt aus der Gruppe bestehend aus Magnesiumsilicat, Kohlenstoff, Silicagel Aluminiumoxid, Titandioxid und Kationentauscherharz.

In einer Ausführungsform des erfindungsgemäßen Verfahrens beträgt das Stoffmengenverhältnis des Sauerstoffs zur zweiten aromatischen Verbindung von 1:500 bis 1:1 bevorzugt von 1:100 bis 1:1. In einer Ausführungsform des erfindungsgemäßen Verfahrens der Katalysator (D) in einer berechneten Menge von 10 ppm bis 15%, bevorzugt von 100 ppm bis 5% und besonders bevorzugt von 100 ppm bis 2% bezogen auf die Menge der zweiten aromatischen Verbindung eingesetzt wird. In einer Ausführungsform des erfindungsgemäßen Verfahrens erfolgt die Herstellung des Pyridin-N-oxids bei einer Temperatur von 0 °C bis 250 °C bevorzugt von 10 °C bis 220 °C und besonders bevorzugt von 20 °C bis 150 °C.

In einer Ausführungsform des erfindungsgemäßen Verfahrens erfolgt die Herstellung des Pyridin-N-oxids bei einem Druck von 1 bara bis 200 bara bevorzugt von 1 bara bis 100 bara und besonders bevorzugt von 1 bara bis 60 bara.

In einer Ausführungsform des erfindungsgemäßen Verfahrens erfolgt die Herstellung des Pyridin-N-oxids in einem Zeitraum von 6 min bis 48 h, bevorzugt von 6 min bis 24 h und besonders bevorzugt von 6 min bis 3 h.

In einer Ausführungsform des erfindungsgemäßen Verfahrens ist das Pyrimidin-N-oxid eine oder mehrere Verbindungen gemäß Formel (VI) und/oder (VII): 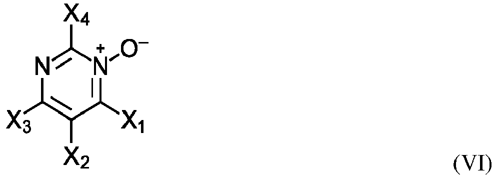 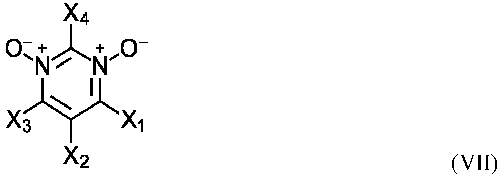 mit
X₁ bis X₄ unabhängig voneinander ausgewählt aus der Gruppe F, Cl, Br, CN, -CO-CF₃, -CO-C(CH3)₃, -CO-CH₂C(CH₃)₃, -CO-C₆H₅, -CO-OC(CH₃)₃, -CO-OCH₂C(CH₃)₃ oder H bevorzugt F, Cl besonders bevorzugt Cl.

In einer Ausführungsform des erfindungsgemäßen Verfahrens das Pyrimidin-N-oxid eine oder mehrere Verbindungen und ausgewählt wird aus der Gruppe bestehend aus 2-Chlorpyrimidin-N-oxid, 2,4-Dichlor-6-methylpyrimidin-1-N-oxid, 2,4-Dichlor-6-methylpyrimidin-3-N-oxid, 2,5-Dichlorpyrimidin-1-N-oxid, 2,5-Dichlorpyrimidin-2-N-oxid.

In einer Ausführungsform des erfindungsgemäßen Verfahrens ist das Pyrimidin-N-oxid erhältlich durch Umsetzung einer dritten aromatischen Verbindung mit Sauerstoff oder einem Sauerstoff-haltigen Gasgemisch in Gegenwart eines Katalysators (E).

In einer Ausführungsform des erfindungsgemäßen Verfahrens wird das Pyrimidin-N-oxid hergestellt durch Umsetzung einer dritten aromatischen Verbindung mit Sauerstoff oder einem Sauerstoff-haltigen Gasgemisch in Gegenwart eines Katalysators (E).

In einer Ausführungsform des erfindungsgemäßen Verfahrens weist die dritte aromatische Verbindung eine Siedetemperatur von 50 °C bis 350 °C bei 1bara auf.

In einer Ausführungsform des erfindungsgemäßen Verfahrens ist die dritte aromatische Verbindung eine oder mehrere Verbindung(en) und wird ausgewählt aus der Gruppe bestehend aus 2-Chlorpyrimidin, 2,4-Dichlor-6-methylpyrimidin, 2,4-Dichlor-6-methylpyrimidin, 2,5-Dichlorpyrimidin, bevorzugt 2,4-Dichlor-6-methylpyrimidin.

In einer Ausführungsform des erfindungsgemäßen Verfahrens ist der Katalysator (E) eine oder mehrere Verbindung(en) und wird ausgewählt wird aus der Gruppe bestehend aus Rutheniumtrichlorid, Titanhaltige Zeolithe und Silber .

In einer Ausführungsform des erfindungsgemäßen Verfahrens beträgt das Stoffmengenverhältnis des Sauerstoffs zur dritten aromatischen Verbindung von 1:500 bis 1:1 bevorzugt von 1:100 bis 1:1.

In einer Ausführungsform des erfindungsgemäßen Verfahrens wird der Katalysator (E) in einer berechneten Menge von 10 ppm bis 15%, bevorzugt von 100 ppm bis 5% und besonders bevorzugt von 100 ppm bis 2% bezogen auf die Menge der dritten aromatischen Verbindung eingesetzt.

In einer Ausführungsform des erfindungsgemäßen Verfahrens erfolgt die Herstellung des Pyrimidin-N-oxid bei einer Temperatur von 0 °C bis 250 °C bevorzugt von 10 °C bis 220 °C und besonders bevorzugt von 20 °C bis 150 °C.

In einer Ausführungsform des erfindungsgemäßen Verfahrens erfolgt die Herstellung des Pyrimidin-N-oxid bei einem Druck von 1 bara bis 200 bara bevorzugt von 1 bara bis 100 bara und besonders bevorzugt von 1 bara bis 60 bara.

In einer Ausführungsform des erfindungsgemäßen Verfahrens erfolgt die Herstellung des Pyrimidin-N-oxid in einem Zeitraum von 6 min bis 48 h, bevorzugt von 6 min bis 24 h und besonders bevorzugt von 6 min bis 3 h.

In einer Ausführungsform des erfindungsgemäßen Verfahrens erfolgt die Herstellung des Alkylenoxids in Gegenwart eines Katalysators (A), wobei der Katalysator (A) ein Metall (A-1) und/oder ein Metallsalz (A-2) umfasst.

In einer Ausführungsform des erfindungsgemäßen Verfahrens ist das Metall (A-1) Kupfer (Cu), Silber (Ag) und/oder Gold (Au) bevorzugt Silber (Ag).

In einer Ausführungsform des erfindungsgemäßen Verfahrens weist das Metallkation des Metallsalzes (A-2) eine Oxidationsstufe von (+I), (+II); (+III) oder (+IV) bevorzugt von (+II); (+III) oder (+IV) auf.

In einer Ausführungsform des erfindungsgemäßen Verfahrens ist das Metallsalz (A-2) ein Nitrat, Halogenid, Tetrafluoroborat, Sulfat, Paratoluolsulfonat, Methansulfonat und/oder Triflat bevorzugt ein Chlorid.

In einer Ausführungsform des erfindungsgemäßen Verfahrens ist das Metallsalz (A-2) eine oder mehrere Verbindung(en) und wird ausgewählt aus der Gruppe bestehend aus Cr₂(SO₄)₃, KCr(SO₄)₂, Cr(NO₃)₃, CrF₃, CrCl₃, FeCl₃, FeBr₃, Eisentriflat, FePO₄, Fe₂(SO4)₃, Fe(NO₃)₃, FeF₃, Eisen-paratoluolsulfonat, CoCl₂, CoBr₂, Co(NO₃)₂, CoBr₂, CoSO₄, CoF₂, Co(BF₄)₂, Co₃(PO₄)₂, CuCl₂, CuSO₄, (CF₃SO₃)₂Cu, CuF₂, Cu(NO₃)₂, Kupfer(II)-pyrophosphat CuCl, CuI und CuBr bevorzugt CrCl₃, FeCl₃, CoCl₂ und CuCl₂. Die Metallsalze können hierbei auch als Hydrate vorliegen.

In einer Ausführungsform des erfindungsgemäßen Verfahrens wird Katalysator (A) in einer berechneten Menge von 10 ppm bis 15%, bevorzugt von 100 ppm bis 5% und besonders bevorzugt von 100 ppm bis 2% bezogen auf die Masse aller eingesetzten Komponenten eingesetzt.

In einer Ausführungsform des erfindungsgemäßen Verfahrens wird der Katalysator (A) auf einem Katalysatorträger (B) aufgebracht unter Bildung eines geträgerten Katalysators (A').

In einer Ausführungsform des erfindungsgemäßen Verfahrens ist der Katalysatorträger (B) ein Metalloxid, ein Erdalkalicarbonat, ein Silicat, ein Siliciumcarbid, ein Siliciumoxycarbid, ein Siliciumnitrid, ein Siliciumoxynitrid und/oder ein Siliciumdioxid.

In einer bevorzugten Ausführungsform des erfindungsgemäßen Verfahrens ist der Katalysatorträger (B) eine oder mehrere Verbindung(en) und wird ausgewählt aus der Gruppe bestehend aus Aluminiumoxid Aluminiumdioxid, Silica, Titandioxid, Zircondioxid, Calciumcarbonat, Phyllosilikat, wie Talkum, Kaolinit und Pyrophyllit und Titandioxid.

In einer Ausführungsform des erfindungsgemäßen Verfahrens wird der Katalysator (A) in einem berechneten Massenanteil von 1,0 -Gew.-% bis 30,0-Gew.-% auf den Katalysatorträger (B) aufgebracht.

In einer Ausführungsform des erfindungsgemäßen Verfahrens wird der Katalysator (A) mithilfe der Nassinfiltrationsmethode (wet-infiltration) oder der incipient-wetness Methode auf den Katalysatorträger (B) aufgebracht.

In einer Ausführungsform des erfindungsgemäßen Verfahrens beträgt das Stoffmengenverhältnis des Alkens zum Arenoxid von 1: 0,01 bis 10:1 bevorzugt von 1:0,1 bis 1:1.

In einer Ausführungsform des erfindungsgemäßen Verfahrens erfolgt die Herstellung des Alkylenoxids in Gegenwart eines Lösungsmittels.

In einer Ausführungsform des erfindungsgemäßen Verfahrens ist das Lösungsmittel eine oder mehrere Verbindung(en) und wird ausgewählt aus der Gruppe bestehend aus CO2, Wasser , Perfluoromethyldecalin, Perfluorodecalin, Perfluoroperhydrophenanthren, Perfluoro(butyltetrahydrofuran), Tetrahydrofuran, 2-Methyl-THF, Essigsäure, Acetonitril, Dimethylsulfoxid, Sulfolan, Aceton, Ethylmethylketon, Dimethylformamid, Dichlormethan, Chloroform, Tetrachlormethan, N-methyl-2-pyrrolidinon, Methyl-t-butylether (MTBE), Dimrthylsulxid (DMSO), Hexamethylphosphoramid, Dichlorbenzol, 1,2-Dichloroethylen, 1,1,1,3,3,3-Hexafluoroisopropanol, Perfluoro-tert-butylalkohol, 1,1,2,3,3-Pentafluoropropan, 1-Brom-2-chlor-1,1,2-trifluoroethan, 1,2-Dichloro-1,1 ,2,3,3,3-hexafluorpropan, Ethylenglycol, Glycerin, und Phenol.

In einer Ausführungsform des erfindungsgemäßen Verfahrens erfolgt die Herstellung bei einer Temperatur von 20 °C bis 200 °C bevorzugt von 50 °C bis 160 °C und besonders bevorzugt von 100 °C bis 150 °C.

In einer Ausführungsform des erfindungsgemäßen Verfahrens erfolgt die Herstellung bei einem Druck von 1 bara bis 200 bara bevorzugt von 1 bara bis 35 bara und besonders bevorzugt von 1 bara bis 28 bara.

In einer Ausführungsform des erfindungsgemäßen Verfahrens erfolgt die Herstellung in einem Zeitraum von 6 min bis 48 h, bevorzugt von 6 min bis 24 h und besonders bevorzugt von 6 min bis 3 h.

In einer Ausführungsform des erfindungsgemäßen Verfahrens beträgt das Stoffmengenverhältnis des Alkens zum Sauerstoff von 1:100 bis 100:1 bevorzugt von 1:30 bis 30:1.

In einer Ausführungsform des erfindungsgemäßen Verfahrens erfolgt die Herstellung in Abwesenheit eines Lösungsmittels.

Hierbei bedeutet erfindungsgemäß ein Herstellungsverfahren in Abwesenheit eines Lösungsmittels, das Lösungsmittelreste beispielsweise infolge der Herstellung der Einsatzstoffe von bis zu 10 Vol.-% bevorzugt von bis zu 5 Vol.-% und besonders bevorzugt von bis 2 Vol.-% bezogen auf die Menge an eingesetztem Propen zugegen sein können.

In einer Ausführungsform des erfindungsgemäßen Verfahrens erfolgt die Herstellung unter Verwendung des unter Verwendung des erfindungsgemäßen geträgerten Katalysators (A').

In einer Ausführungsform des erfindungsgemäßen Verfahrens erfolgt die Herstellung bei einer Temperatur von 20 °C bis 500 °C bevorzugt von 50 °C bis 400 °C und besonders bevorzugt von 50 °C bis 250 °C.

In einer Ausführungsform des erfindungsgemäßen Verfahrens erfolgt die Herstellung bei einem Druck von 1 bara bis 200 bara bevorzugt von 2 bara bis 100 bara und besonders bevorzugt von 2 bara bis 50 bara.

In einer Ausführungsform des erfindungsgemäßen Verfahrens erfolgt die Herstellung mit einer Raumgeschwindigkeit von 100 h-1 bis 10000 h-1, bevorzugt von 200 h-1 bis 5000 h-1 und besonders bevorzugt von 500 h-1 bis 2000 h-1.

In einer Ausführungsform des erfindungsgemäßen Verfahrens beträgt das Stoffmengenverhältnis des Alkens zum Sauerstoff von 1,0:0,1 bis 2,0:1,0 bevorzugt von 1,0:0,5 bis 2,0:1,0 und besonders bevorzugt von 1,0:0,8 bis 2,0:1,0.

In einer Ausführungsform des erfindungsgemäßen Verfahrens wird eine erste aromatische Verbindung durch Umsetzung des Alkens mit dem Arenoxid, eine zweite aromatische Verbindung durch Umsetzung des Alkens mit dem Pyridin-N-oxids und/oder eine dritte aromatische Verbindung durch Umsetzung des Alkens mit dem Pyrimidin-N-oxid gebildet.

In einer Ausführungsform des erfindungsgemäßen Verfahrens wird das Alken kontinuierlich oder stufenweise bevorzugt kontinuierlich in den ersten Reaktor dosiert.

In einer Ausführungsform des erfindungsgemäßen Verfahrens wird das Arenoxid, Pyridin-N-oxid und/oder das Pyrimidin-N-oxid kontinuierlich oder stufenweise bevorzugt kontinuierlich in den ersten Reaktor dosiert.

In einer Ausführungsform des erfindungsgemäßen Verfahrens wird das Alken und das Arenoxid, Pyridin-N-oxid und/oder das Pyrimidin-N-oxid kontinuierlich oder stufenweise bevorzugt kontinuierlich in den ersten Reaktor dosiert.

In einer Ausführungsform des erfindungsgemäßen Verfahrens wird das Alkylenoxid kontinuierlich oder stufenweise bevorzugt kontinuierlich aus dem ersten Reaktor entnommen.

In einer Ausführungsform des erfindungsgemäßen Verfahrens wird die erste aromatische Verbindung, die zweite aromatische Verbindung und/oder die dritte aromatische Verbindung kontinuierlich oder stufenweise bevorzugt kontinuierlich aus dem ersten Reaktor entnommen.

In einer Ausführungsform des erfindungsgemäßen Verfahrens wird das Alkylenoxid und die halogenierte, bevorzugt das Alkylenoxid und die erste aromatische Verbindung, die zweite aromatische Verbindung und/oder die dritte aromatische Verbindung kontinuierlich oder stufenweise bevorzugt kontinuierlich aus dem ersten Reaktor entnommen.

In einer Ausführungsform des erfindungsgemäßen Verfahrens wird der Katalysator (A) kontinuierlich oder stufenweise bevorzugt kontinuierlich in den Reaktor dosiert.

In einer Ausführungsform des erfindungsgemäßen Verfahrens ist der erste Reaktor ein Rührkessel, Strömungsrohr, Blasensäule, Schlaufenreaktor, Rieselbett-Reaktor, Sprühturm-Reaktor oder Fallfilm-Reaktor.

In einer Ausführungsform des erfindungsgemäßen Verfahrens wird das Arenoxid, das Pyridinoxid und/oder das Pyrimidin-N-oxid in einem zweiten Reaktor hergestellt, wobei der zweite Reaktor ungleich dem ersten Reaktor ist.

In einer Ausführungsform des erfindungsgemäßen Verfahrens erfolgt die Herstellung des Arenoxids, des Pyridin-N-oxids und/oder das Pyrimidin-N-oxids im zweiten Reaktor bei einer Temperatur von 20 °C bis 250 °C bevorzugt von 50 °C bis 220 °C und besonders bevorzugt von 100 °C bis 200 °C.

In einer Ausführungsform des erfindungsgemäßen Verfahrens erfolgt die Herstellung des Arenoxids, des Pyridin-N-oxids und/oder das Pyrimidin-N-oxids im zweiten Reaktor bei einem Druck von 1 bara bis 200 bara bevorzugt von 1 bara bis 100 bara und besonders bevorzugt von 1 bara bis 60 bara.

In einer Ausführungsform des erfindungsgemäßen Verfahrens erfolgt die Herstellung des Arenoxids, des Pyridin-N-oxids und/oder das Pyrimidin-N-oxids im zweiten Reaktor in einem Zeitraum von 6 min bis 48 h, bevorzugt von 6 min bis 24 h und besonders bevorzugt von 6 min bis 3 h.

In einer Ausführungsform des erfindungsgemäßen Verfahrens wird die erste aromatische Verbindung, die zweite aromatische Verbindung und/oder die dritte aromatische Verbindung kontinuierlich oder stufenweise bevorzugt kontinuierlich in den zweiten Reaktor dosiert.

In einer Ausführungsform des erfindungsgemäßen Verfahrens wird Sauerstoff oder das Sauerstoff-haltige Gasgemisch kontinuierlich oder stufenweise bevorzugt kontinuierlich in den zweiten Reaktor dosiert.

In einer Ausführungsform des erfindungsgemäßen Verfahrens wird die erste aromatische Verbindung, die zweite aromatische Verbindung und/oder die dritte aromatische Verbindung und Sauerstoff oder das Sauerstoff-haltige Gasgemisch kontinuierlich oder stufenweise bevorzugt kontinuierlich in den zweiten Reaktor dosiert.

In einer Ausführungsform des erfindungsgemäßen Verfahrens wird das Arenoxid, das Pyridin-N-oxid und/oder das Pyrimidin-N-oxid kontinuierlich oder stufenweise bevorzugt kontinuierlich aus dem zweiten Reaktor entnommen.

In einer Ausführungsform des erfindungsgemäßen Verfahrens wird der Katalysator (C) kontinuierlich oder stufenweise bevorzugt kontinuierlich in den zweiten Reaktor dosiert.

In einer Ausführungsform des erfindungsgemäßen Verfahrens ist der zweite Reaktor ein Rührkessel, Strömungsrohr, Blasensäule, Schlaufenreaktor, Rieselbett-Reaktor, Sprühturm-Reaktor oder Fallfilm-Reaktor.

In einer Ausführungsform des erfindungsgemäßen Verfahrens wird das im zweiten Reaktor hergestellte Arenoxid, Pyridin-N-oxid und/oder Pyrimidin-N-oxid kontinuierlich oder stufenweise bevorzugt kontinuierlich in ersten Reaktor dosiert.

Ein weiterer Gegenstand der Erfindung ist die Verwendung des Katalysators (A) zur Herstellung des erfindungsgemäßen Alkylenoxids durch Umsetzung des erfindungsgemäßen Alkens mit dem erfindungsgemäßen Arenoxid, dem erfindungsgemäßen Pyridin-N-oxids und/oder dem erfindungsgemäßen Pyrimidin-N-oxids bevorzugt mit dem erfindungsgemäßen Arenoxid und/oder dem erfindungsgemäßen Pyridin-N-oxid.

In einer ersten Ausführung betrifft die Erfindung ein Verfahren zur Herstellung eines Alkylenoxids durch Umsetzung eines Alkens mit einem Arenoxid, Pyridin-N-oxid und/oder Pyrimidin-N-oxid bevorzugt mit einem Arenoxid und/oder Pyridin-N-oxid bevorzugt in Gegenwart eines Katalysators (A) in einem ersten Reaktor, wobei der Katalysator (A) ein Metall (A-1) und/oder ein Metallsalz (A-2) umfasst, wobei das Metall (A-1) Kupfer, Silber und/oder Gold ist, wobei das Metallsalz (A-2) Chrom (Cr), Eisen (Fe), Cobalt (Co), und/oder Kupfer (Cu) -Kation(en) umfasst, und wobei die Umsetzung in Abwesenheit von Sauerstoff oder einem Sauerstoff-haltigen Gasgemisch erfolgt.

In einer zweiten Ausführung betrifft die Erfindung ein Verfahren gemäß der ersten Ausführung, wobei das Alkylenoxid eine oder mehrere Verbindung(en) ist und ausgewählt wird aus der Gruppe bestehend aus Ethylenoxid, Propylenoxid, 1,2-Butylenoxid, 1,2-Pentylenoxid, 1,2-Hexylenoxid, 1,2-Heptylenoxid und 1,2-Octylenoxid bevorzugt Ethylenoxid und Propylenoxid besonders bevorzugt Propylenoxid.

In einer dritten Ausführung betrifft die Erfindung ein Verfahren gemäß der ersten oder zweiten Ausführungsform, wobei das Arenoxid eine oder mehrere Verbindungen gemäß Formel (I), (II), (III) und/oder (IV) ist: 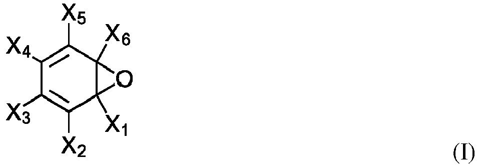 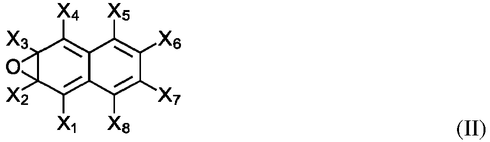 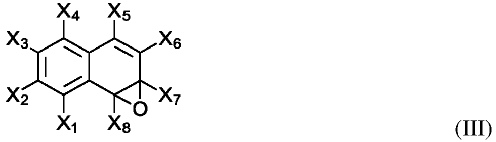 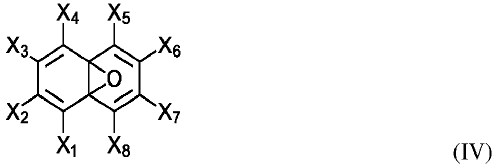 mit X₁ bis X₈ unabhängig voneinander ausgewählt aus der Gruppe von F, Cl, Br, CN, -CO-RCF₃, -CO-C(CH₃)₃, -CO-CH₂C(CH₃)₃, -CO-C₆H₅, -CO-OC(CH₃)₃, -CO-OCH₂C(CH₃)₃ oder H bevorzugt F, Cl besonders bevorzugt Cl.

In einer vierten Ausführung betrifft die Erfindung ein Verfahren gemäß einer der der ersten oder vierten Ausführungsform, wobei das Arenoxid eine oder mehrere Verbindung(en) ist und ausgewählt wird aus der Gruppe bestehend aus Hexafluorbenzoloxid, Hexachlorbenzoloxid, 1-Brom-2,3,4-trifluorbenzoloxid, Pentafluorbenzoloxid, 1,3,5-Trichlor-2,4,6-trifluorbenzoloxid, 1,3,5-Trfluorbenzoloxid, 1,2-Dibrom-3,5-difluorbenzoloxid, 1,2,4,5-Tetrafluorbenzoloxid, Brompentafluorbenzoloxid, 1,3,5-Trichlorbenzoloxid, 1-Brom-3,5-dichlrobenzoloxid, Orthodichlorbenzoloxid, 1,2,4,5-Tetrachlorbenzoloxid, 1,2,3-Trichlorbenzoloxidund 1,5-Dichlro-2-fluorbenzoloxid, bevorzugt Hexafluorbenzoloxid, und Hexachlorbenzoloxid.

In einer fünften Ausführung betrifft die Erfindung ein Verfahren gemäß einer der ersten bis vierten Ausführungsform, wobei das Arenoxid erhältlich ist durch Umsetzung einer ersten aromatischen Verbindung mit Sauerstoff oder einem Sauerstoff-haltigen Gasgemisch in Gegenwart eines Katalysators (C).

In einer sechsten Ausführung betrifft die Erfindung ein Verfahren gemäß einer der ersten bis fünften Ausführungsform, wobei das Arenoxid hergestellt wird durch Umsetzung einer ersten aromatischen Verbindung mit Sauerstoff oder einem Sauerstoff-haltigen Gasgemisch in Gegenwart eines Katalysators (C).

In einer siebten Ausführung betrifft die Erfindung ein Verfahren gemäß der fünften oder sechsten Ausführungsform, wobei die erste aromatische Verbindung eine Siedetemperatur von 50 °C bis 350 °C bei 1bara aufweist.

In einer achten Ausführung betrifft die Erfindung ein Verfahren gemäß einer der fünften bis siebten Ausführungsform, wobei die erste aromatische Verbindung eine oder mehrere Verbindung(en) ist und ausgewählt wird aus der Gruppe bestehend aus Hexafluorbenzol, Hexachlorbenzol, 1-Brom-2,3,4-trifluorbenzol, Pentafluorbenzol, 1,3,5-Trichlor-2,4,6-trifluorbenzol, 1,3,5-Trifluorbenzol, 1,2-Dibrom-3,5-difluorbenzol, 1,2,4,5-Tetrafluorbenzol, Brompentafluorbenzol, 1,3,5-Trichlorbenzol, 1-Brom-3,5-dichlrobenzol, Orthodichlorbenzol, 1,2,4,5-Tetrachlorbenzol, 1,2,3-Trichlorbenzol und 1,5-Dichlro-2-fluorbenzol bevorzugt Hexafluorbenzol und Hexachlorbenzol.

In einer neunten Ausführung betrifft die Erfindung ein Verfahren gemäß einer der fünften bis achten Ausführungsform, wobei der Katalysator (C) eine oder mehrere Verbindung(en) ist und ausgewählt wird aus der Gruppe bestehend aus Silber, Silber geträgert auf Magnesiumsilikat und Enzym Cytochrom P450.

In einer zehnten Ausführung betrifft die Erfindung ein Verfahren gemäß einer der fünften bis neunten Ausführungsform, wobei das Stoffmengenverhältnis des Sauerstoffs zur ersten aromatischen Verbindung von 1:500 bis 1:1 bevorzugt von 1:100 bis 1:1 beträgt.

In einer elften Ausführung betrifft die Erfindung ein Verfahren gemäß einer der fünften bis zehnten Ausführungsform, wobei der Katalysator (C) in einer berechneten Menge von 10 ppm bis 15%, bevorzugt von 100 ppm bis 5% und besonders bevorzugt von 100 ppm bis 2% bezogen auf die Menge der ersten aromatischen Verbindung eingesetzt wird.

In einer zwölften Ausführung betrifft die Erfindung ein Verfahren gemäß einer der fünften bis elften Ausführungsform, wobei die Herstellung des Arenoxids bei einer Temperatur von 20 °C bis 250 °C bevorzugt von 50 °C bis 220 °C und besonders bevorzugt von 100 °C bis 200 °C erfolgt.

In einer dreizehnten Ausführung betrifft die Erfindung ein Verfahren gemäß einer der fünften bis zwölften Ausführungsform, wobei die Herstellung des Arenoxids bei einem Druck von 1 bara bis 200 bara bevorzugt von 1 bara bis 100 bara und besonders bevorzugt von 1 bara bis 60 bara erfolgt. In einer vierzehnten Ausführung betrifft die Erfindung ein Verfahren gemäß einer der fünften bis dreizehnten Ausführungsform, wobei die Herstellung des Arenoxids in einem Zeitraum von 6 min bis 48 h, bevorzugt von 6 min bis 24 h und besonders bevorzugt von 6 min bis 3 h erfolgt.

In einer fünfzehnten Ausführung betrifft die Erfindung ein Verfahren gemäß einer der ersten bis vierzehnten Ausführungsform, wobei das Pyridin-N-oxids eine oder mehrere Verbindungen gemäß Formel (V) ist: 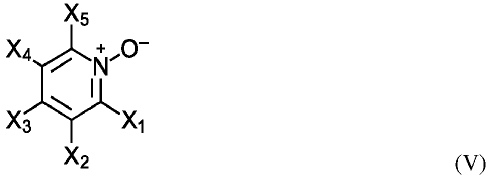 mit
E ausgewählt aus der Gruppe von N
X₁ bis X₅ unabhängig voneinander ausgewählt aus der Gruppe von F, Cl, Br, CN, -CO-CF₃, -CO-C(CH₃)₃, -CO-CH₂C(CH₃)₃, -CO-C₆H₅, -CO-OC(CH₃)₃, -CO-OCH₂C(CH₃)₃ oder H bevorzugt F, Cl besonders bevorzugt Cl.

In einer sechzehnten Ausführung betrifft die Erfindung ein Verfahren gemäß einer der ersten bis fünfzehnten Ausführungsform, wobei das Pyridin-N-oxid eine oder mehrere Verbindung(en) ist und ausgewählt wird aus der Gruppe bestehend aus Pentafluorpyridin-N-Oxid, 2-Brom-3,5-dichlorpyridin-N-oxid, 3-Chlorpyridin-N-oxid, 3,6-Dichlorpyridin-N-oxid, 3,5-Dichlorpyridin-N-oxid, 3-Chlor-2,5,6-trifluorpyridin-N-oxid, 3-Chlo-2,4,5,6-tetrafluorpyridin-1-N-oxid, 3-Chlo-2,4,5,6-tetrafluorpyridin-3-N-oxid, bevorzugt Pentafluorpyridin-N-Oxid.

In einer siebzehnten Ausführung betrifft die Erfindung ein Verfahren gemäß der fünfzehnten oder sechzehnten Ausführungsform, wobei das Pyridin-N-oxid erhältlich ist durch Umsetzung einer zweiten aromatischen Verbindung mit Sauerstoff oder einem Sauerstoff-haltigen Gasgemisch in Gegenwart eines Katalysators (D).

In einer achtzehnten Ausführung betrifft die Erfindung ein Verfahren gemäß der fünfzehnten oder sechzehnten Ausführungsform, wobei das Pyridin-N-oxid hergestellt wird durch Umsetzung einer zweiten aromatischen Verbindung mit Sauerstoff oder einem Sauerstoff-haltigen Gasgemisch in Gegenwart eines Katalysators (D).

In einer neunzehnten Ausführung betrifft die Erfindung ein Verfahren gemäß der siebzehnten oder achtzehnten Ausführungsform, wobei die zweite aromatische Verbindung eine Siedetemperatur von 50 °C bis 350 °C bei 1bara aufweist.

In einer zwanzigsten Ausführung betrifft die Erfindung ein Verfahren gemäß einer der siebzehnten bis neunzehnten Ausführungsform, wobei die zweite aromatische Verbindung eine oder mehrere Verbindung(en) ist und ausgewählt wird aus der Gruppe bestehend aus Pentafluorpyridin-N-Oxid, 2-Brom-3,5-dichlorpyridin, 3-Chlorpyridin, 3,6-Dichlorpyridin, 3,5-Dichlorpyridin, 3-Chlor-2,5,6-trifluorpyridin, 3-Chlo-2,4,5,6-tetrafluorpyridin, 3-Chlo-2,4,5,6-tetrafluorpyridin, bevorzugt Pentafluorpyridin.

In einer einundzwanzigsten Ausführung betrifft die Erfindung ein Verfahren gemäß einer der siebzehnten bis zwanzigsten Ausführungsform, wobei der Katalysator (D) eine oder mehrere Verbindung(en) ist und ausgewählt wird aus der Gruppe bestehend aus Rutheniumtrichlorid, Silber und Titan-haltige Zeolithe.

In einer zweiundzwanzigsten Ausführung betrifft die Erfindung ein Verfahren gemäß der einundzwanzigsten Ausführungsform, wobei der Katalysator (D) auf einem Katalysatorträger aufgebracht wird, und der Katalysatorträger eine oder mehrere ist und ausgewählt wird aus der Gruppe bestehend aus Magnesiumsilicat, Silicagel Aluminiumoxid, Titandioxid und Kationentauscherharz .

In einer dreiundzwanzigsten Ausführung betrifft die Erfindung ein Verfahren gemäß einer der siebzehnten bis zweiundzwanzigsten Ausführungsform, wobei das Stoffmengenverhältnis des Sauerstoffs zur zweiten aromatischen Verbindung von 1:500 bis 1:1 bevorzugt von 1:100 bis 1:1 beträgt.

In einer vierundzwanzigsten Ausführung betrifft die Erfindung ein Verfahren gemäß einer der siebzehnten bis dreiundzwanzigsten, wobei der Katalysator (D) in einer berechneten Menge von 10 ppm bis 15%, bevorzugt von 100 ppm bis 5% und besonders bevorzugt von 100 ppm bis 2% bezogen auf die Menge der zweiten aromatischen Verbindung eingesetzt wird.

In einer fünfundzwanzigsten Ausführung betrifft die Erfindung ein Verfahren gemäß einer der siebzehnten bis dreiundzwanzigsten, wobei die Herstellung des Pyridin-N-oxids bei einer Temperatur von 0 °C bis 250 °C bevorzugt von 10 °C bis 220 °C und besonders bevorzugt von 20 °C bis 150 °C erfolgt.

In einer sechsundzwanzigsten Ausführung betrifft die Erfindung ein Verfahren gemäß einer der siebzehnten bis fünfundzwanzigsten, wobei die Herstellung des Pyridin-N-oxids bei einem Druck von 1 bara bis 200 bara bevorzugt von 1 bara bis 100 bara und besonders bevorzugt von 1 bara bis 60 bara erfolgt.

In einer siebenundzwanzigsten Ausführung betrifft die Erfindung ein Verfahren gemäß einer der siebzehnten bis sechsundzwanzigsten, wobei die Herstellung des Pyridin-N-oxids in einem Zeitraum von 6 min bis 48 h, bevorzugt von 6 min bis 24 h und besonders bevorzugt von 6 min bis 3 h erfolgt. In einer achtundzwanzigsten Ausführung betrifft die Erfindung ein Verfahren gemäß einer der ersten bis siebenundzwanzigsten, wobei das Pyrimidin-N-oxid eine oder mehrere Verbindungen gemäß Formel (VI) ist: 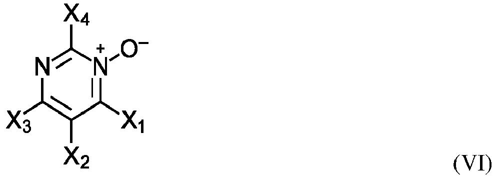 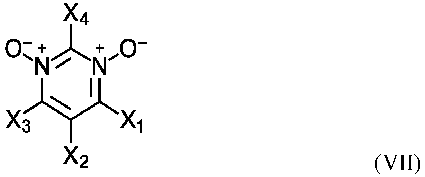 mit
X₁ bis X₄ unabhängig voneinander ausgewählt aus der Gruppe von F, Cl, Br, CN, -CO-CF₃, -CO-C(CH₃)₃, -CO-CH₂C(CH₃)₃, -CO-C₆H₅, -CO-OC(CH₃)₃, -CO-OCH₂C(CH₃)₃ oder H bevorzugt F, Cl besonders bevorzugt Cl.

In einer neunundzwanzigsten Ausführung betrifft die Erfindung ein Verfahren gemäß einer der ersten bis achtundzwanzigsten Ausführungsform, wobei das Pyrimidin-N-oxid eine oder mehrere Verbindungen und ausgewählt wird aus der Gruppe bestehend aus 2-Chlorpyrimidin-N-oxid, 2,4-Dichlor-6-methylpyrimidin-1-N-oxid, 2,4-Dichlor-6-methylpyrimidin-3-N-oxid, 2,5-Dichlorpyrimidin-1-N-oxid, 2,5-Dichlorpyrimidin-2-N-oxid.

In einer dreißigsten Ausführung betrifft die Erfindung ein Verfahren gemäß einer der ersten bis neunundzwanzigsten Ausführungsform, wobei das Pyrimidin-N-oxid erhältlich ist durch Umsetzung einer dritten aromatischen Verbindung mit Sauerstoff oder einem Sauerstoff-haltigen Gasgemisch in Gegenwart eines Katalysators (E).

In einer einunddreißigsten Ausführung betrifft die Erfindung ein Verfahren gemäß einer der ersten bis dreißigsten Ausführungsform, wobei das Pyrimidin-N-oxid hergestellt wird durch Umsetzung einer dritten aromatischen Verbindung mit Sauerstoff oder einem Sauerstoff-haltigen Gasgemisch in Gegenwart eines Katalysators (E).

In einer zweiunddreißigsten Ausführung betrifft die Erfindung ein Verfahren gemäß der dreißigsten oder einunddreißigsten Ausführungsform, wobei die dritte aromatische Verbindung eine Siedetemperatur von 50 °C bis 350 °C bei 1bara aufweist.

In einer dreiunddreißigsten Ausführung betrifft die Erfindung ein Verfahren gemäß einer der dreißigsten bis zweiunddreißigsten Ausführungsform, wobei die dritte aromatische Verbindung eine oder mehrere Verbindung(en) ist und ausgewählt wird aus der Gruppe bestehend aus 2-Chlorpyrimidin, 2,4-Dichlor-6-methylpyrimidin, 2,4-Dichlor-6-methylpyrimidin, 2,5-Dichlorpyrimidin, bevorzugt 2,4-Dichlor-6-methylpyrimidin.

In einer vierunddreißigsten Ausführung betrifft die Erfindung ein gemäß einer der dreißigsten bis dreiunddreißigsten Ausführungsform, wobei der Katalysator (E) eine oder mehrere Verbindung(en) ist und ausgewählt wird aus der Gruppe bestehend aus Rutheniumtrichlorid, Titan-haltige Zeolithe und Silber.

In einer fünfunddreißigsten Ausführung betrifft die Erfindung ein Verfahren gemäß einer der dreißigsten bis vierunddreißigsten Ausführungsform, wobei das Stoffmengenverhältnis des Sauerstoffs zur dritten aromatischen Verbindung von 1:500 bis 1:1 bevorzugt von 1:100 bis 1:1 beträgt.

In einer sechsunddreißigsten Ausführung betrifft die Erfindung ein Verfahren gemäß einer der dreißigsten bis fünfunddreißigsten Ausführungsform, wobei der Katalysator (E) in einer berechneten Menge von 10 ppm bis 15%, bevorzugt von 100 ppm bis 5% und besonders bevorzugt von 100 ppm bis 2% bezogen auf die Menge der dritten aromatischen Verbindung eingesetzt wird.

In einer siebenunddreißigsten Ausführung betrifft die Erfindung ein Verfahren gemäß einer der dreißigsten bis sechsunddreißigsten Ausführungsform, wobei die Herstellung des Pyrimidin-N-oxid bei einer Temperatur von 0 °C bis 250 °C bevorzugt von 10 °C bis 220 °C und besonders bevorzugt von 20 °C bis 150 °C erfolgt.

In einer achtunddreißigsten Ausführung betrifft die Erfindung ein Verfahren gemäß einer der dreißigsten bis siebenunddreißigsten Ausführungsform, wobei die Herstellung des Pyrimidin-N-oxid bei einem Druck von 1 bara bis 200 bara bevorzugt von 1 bara bis 100 bara und besonders bevorzugt von 1 bara bis 60 bara erfolgt.

In einer neununddreißigsten Ausführung betrifft die Erfindung ein Verfahren gemäß einer der dreißigsten bis achtunddreißigsten Ausführungsform, wobei die Herstellung des Pyrimidin-N-oxid in einem Zeitraum von 6 min bis 48 h, bevorzugt von 6 min bis 24 h und besonders bevorzugt von 6 min bis 3 h erfolgt.

In einer vierzigsten Ausführung betrifft die Erfindung ein Verfahren gemäß einer der ersten bis neununddreißigsten Ausführungsform, wobei der Metall (A-1) Kupfer (Cu), Silber (Ag) und/oder Gold (Au) bevorzugt Silber (Ag) ist.

In einer einundvierzigsten Ausführung betrifft die Erfindung ein Verfahren gemäß einer der ersten bis vierzigsten Ausführungsform, wobei das Metallkation des Metallsalzes (A-2) eine Oxidationsstufe von (+I); (+II); (+III) oder (+IV) bevorzugt von (+II); (+III) oder (+IV) aufweist. In einer zweiundvierzigsten Ausführung betrifft die Erfindung ein Verfahren gemäß einer der ersten bis einundvierzigsten Ausführungsform, wobei das Metallsalz (A-2) ein Nitrat, Halogenid, Tetrafluoroborat, Sulfat, Paratoluolsulfonat, Methansulfonat und/oder Triflat bevorzugt ein Chlorid ist.

In einer dreiundvierzigsten Ausführung betrifft die Erfindung ein Verfahren gemäß einer der ersten bis zweiundvierzigsten Ausführungsform, wobei das Metallsalz (A-2) eine oder mehrere Verbindung(en) ist und ausgewählt wird aus der Gruppe bestehend aus Cr₂(SO₄)₃, KCr(SO₄)₂, Cr(NO₃)₃, CrF₃, CrCl₃, FeCl₃, FeBr₃, Eisentriflat, FePO₄, Fe₂(SO4)₃, Fe(NO₃)₃, FeF₃, Eisen-paratoluolsulfonat, CoCl₂, CoBr₂, Co(NO₃)₂, CoBr₂, CoSO₄, CoF₂, Co(BF₄)₂, Co₃(PO₄)₂, CuCl₂, CuSO₄, (CF₃SO₃)₂Cu, CuF₂, Cu(NO₃)₂, Kupfer(II)-pyrophosphat, und CuCl, CuI, CuBr bevorzugt CrCl₃, FeCl₃, CoCl₂ und CuCl₂.

In einer vierundvierzigsten Ausführung betrifft die Erfindung ein Verfahren gemäß einer der ersten bis dreiundvierzigsten Ausführungsform, wobei Katalysator (A) in einer berechneten Menge von 10 ppm bis 15%, bevorzugt von 100 ppm bis 5% und besonders bevorzugt von 100 ppm bis 2% bezogen auf die Masse aller eingesetzten Komponenten eingesetzt wird.

In einer fünfundvierzigsten Ausführung betrifft die Erfindung ein Verfahren gemäß einer der ersten bis vierundvierzigsten Ausführungsform, wobei der Katalysator (A) auf einem Katalysatorträger (B) aufgebracht wird unter Bildung eines geträgerten Katalysators (A').

In einer sechsundvierzigsten Ausführung betrifft die Erfindung ein Verfahren gemäß der fünfundvierzigsten Ausführungsform, wobei der Katalysatorträger (B) ein Metalloxid, ein Erdalkalicarbonat, ein Silicat, ein Siliciumcarbid, ein Siliciumoxycarbid, ein Siliciumnitrid, ein Siliciumoxynitrid und/oder ein Siliciumdioxid ist.

In einer siebenundvierzigsten Ausführung betrifft die Erfindung ein Verfahren gemäß der fünfundvierzigsten oder sechsundvierzigsten Ausführungsform, wobei der Katalysatorträger (B) eine oder mehrere Verbindung(en) ist und ausgewählt wird aus der Gruppe bestehend aus Aluminiumoxid Aluminiumdioxid, Silica, Titandioxid, Zircondioxid, Calciumcarbonat, Phyllosilikat, wie Talkum, Kaolinit und Pyrophyllit und Titandioxid.

In einer achtundvierzigsten Ausführung betrifft die Erfindung ein Verfahren gemäß einer der fünfundvierzigsten bis siebenundvierzigsten Ausführungsform, wobei der Katalysator (A) in einem berechneten Massenanteil von 1,0 -Gew.-% bis 30,0-Gew.-% auf den Katalysatorträger (B) aufgebracht wird.

In einer neunundvierzigsten Ausführung betrifft die Erfindung ein Verfahren gemäß einer der fünfundvierzigsten bis achtundvierzigsten Ausführungsform, wobei der Katalysator (A) mithilfe der Nassinfiltrationsmethode (wet-infiltration) oder der incipient-wetness Methode auf den Katalysatorträger (B) unter Bildung des geträgerten Katalysators (A'). aufgebracht wird.

In einer fünfzigsten Ausführung betrifft die Erfindung ein Verfahren gemäß einer der ersten bis neunundvierzigsten Ausführungsform, wobei das Stoffmengenverhältnis des Alkens zum Arenoxid von 1: 0,01 bis 10:1 bevorzugt von 1:0,1 bis 1:1 beträgt.

In einer einundfünfzigsten Ausführung betrifft die Erfindung ein Verfahren gemäß einer der ersten bis fünfzigsten Ausführungsform, wobei die Herstellung des Alkylenoxids in Gegenwart eines Lösungsmittels erfolgt.

In einer zweiundfünfzigsten Ausführung betrifft die Erfindung ein Verfahren gemäß der einundfünfzigsten Ausführungsform, wobei das Lösungsmittel eine oder mehrere Verbindung(en) ist und ausgewählt wird aus der Gruppe bestehend aus CO2, Wasser , Perfluoromethyldecalin, Perfluorodecalin, Perfluoroperhydrophenanthren, Perfluoro(butyltetrahydrofuran), Tetrahydrofuran, 2-Methyl-THF, Essigsäure, Acetonitril, Dimethylsulfoxid, Sulfolan, Aceton, Ethylmethylketon, Dimethylformamid, Dichlormethan, Chloroform, Tetrachlormethan, N-methyl-2-pyrrolidinon, Methyl-t-butylether (MTBE), Dimrthylsulxid (DMSO), Hexamethylphosphoramid, Dichlorbenzol, 1,2-Dichloroethylen, 1,1,1,3,3,3-Hexafluoroisopropanol, Perfluoro-tert-butylalkohol, 1,1,2,3,3-Pentafluoropropan, 1-Brom-2-chlor-1,1,2-trifluoroethan, 1,2-Dichloro-1,1,2,3,3,3-hexafluorpropan, Ethylenglycol, Glycerin, und Phenol.

In einer dreiundfünfzigsten Ausführung betrifft die Erfindung ein Verfahren gemäß der einundfünfzigsten oder zweiundfünfzigsten Ausführungsform, wobei die Herstellung bei einer Temperatur von 20 °C bis 200 °C bevorzugt von 50 °C bis 160 °C und besonders bevorzugt von 100 °C bis 150 °C erfolgt.

In einer vierundfünfzigsten Ausführung betrifft die Erfindung ein Verfahren gemäß einer der einundfünfzigsten bis dreiundfünfzigsten Ausführungsform, wobei die Herstellung bei einem Druck von 1 bara bis 200 bara bevorzugt von 1 bara bis 35 bara und besonders bevorzugt von 1 bara bis 28 bara erfolgt.

In einer fünfundfünfzigsten Ausführung betrifft die Erfindung ein Verfahren gemäß einer der einundfünfzigsten bis vierundfünfzigsten Ausführungsform, wobei die Herstellung in einem Zeitraum von 6 min bis 48 h, bevorzugt von 6 min bis 24 h und besonders bevorzugt von 6 min bis 3 h erfolgt.

In einer sechsundfünfzigsten Ausführung betrifft die Erfindung ein Verfahren gemäß einer der einundfünfzigsten bis fünfundfünfzigsten Ausführungsform, wobei das Stoffmengenverhältnis des Alkens zum Sauerstoff von 1:100 bis 100:1 bevorzugt von 1:30 bis 30:1 beträgt.

In einer siebenundfünfzigsten Ausführung betrifft die Erfindung ein Verfahren gemäß einer der ersten bis sechsundfünfzigsten Ausführungsform, wobei die Herstellung in Abwesenheit eines Lösungsmittels erfolgt.

In einer achtundfünfzigsten Ausführung betrifft die Erfindung ein Verfahren gemäß der siebenundfünfzigsten Ausführungsform, wobei die Herstellung unter Verwendung geträgerten Katalysators (A') gemäß einer der fünfundvierzigsten bis neunundvierzigsten Ausführungsform.

In einer neunundfünfzigsten Ausführung betrifft die Erfindung ein Verfahren gemäß der siebenundfünfzigsten oder achtundfünfzigsten Ausführungsform, wobei die Herstellung bei einer Temperatur von 20 °C bis 500 °C bevorzugt von 50 °C bis 400 °C und besonders bevorzugt von 50 °C bis 250 °C erfolgt.

In einer sechzigsten Ausführung betrifft die Erfindung ein Verfahren gemäß einer der siebenundfünfzigsten bis neunundfünfzigsten Ausführungsform, wobei die Herstellung bei einem Druck von 1 bara bis 200 bara bevorzugt von 2 bara bis 100 bara und besonders bevorzugt von 2 bara bis 50 bara erfolgt.

In einer einundsechzigsten Ausführung betrifft die Erfindung ein Verfahren gemäß einer der siebenundfünfzigsten bis sechzigsten Ausführungsform, wobei die Herstellung mit einer Raumgeschwindigkeit von 100 h⁻¹ bis 10000 h⁻¹, bevorzugt von 200 h⁻¹ bis 5000 h⁻¹ und besonders bevorzugt von 500 h⁻¹ bis 2000 h⁻¹ erfolgt.

In einer zweiundsechzigsten Ausführung betrifft die Erfindung ein Verfahren gemäß einer der siebenundfünfzigsten bis einundsechzigsten Ausführungsform, wobei das Stoffmengenverhältnis des Alkens zum Sauerstoff von 1,0:0,1 bis 2,0:1,0 bevorzugt von 1,0:0,5 bis 2,0:1,0 und besonders bevorzugt von 1,0:0,8 bis 2,0:1,0 beträgt.

In einer dreiundsechzigsten Ausführung betrifft die Erfindung ein Verfahren gemäß einer der ersten bis zweiundsechzigsten Ausführungsform, wobei eine erste aromatische Verbindung durch Umsetzung des Alkens mit dem Arenoxid, eine zweite aromatische Verbindung durch Umsetzung des Alkens mit dem Pyridin-N-oxids und/oder eine dritte aromatische Verbindung durch Umsetzung des Alkens mit dem Pyrimidin-N-oxid gebildet wird.

In einer vierundsechzigsten Ausführung betrifft die Erfindung ein Verfahren gemäß einer der ersten bis dreiundsechzigsten Ausführungsform, wobei das Alken kontinuierlich oder stufenweise bevorzugt kontinuierlich in den ersten Reaktor dosiert wird.

In einer fünfundsechzigsten Ausführung betrifft die Erfindung ein Verfahren gemäß einer der ersten bis vierundsechzigsten Ausführungsform, wobei das Arenoxid, Pyridin-N-oxid und/oder das Pyrimidin-N-oxid kontinuierlich oder stufenweise bevorzugt kontinuierlich in den ersten Reaktor dosiert wird.

In einer sechsundsechzigsten Ausführung betrifft die Erfindung ein Verfahren gemäß einer der ersten bis fünfundsechzigsten Ausführungsform, wobei das Alken und das Arenoxid, Pyridin-N-oxid und/oder das Pyrimidin-N-oxid kontinuierlich oder stufenweise bevorzugt kontinuierlich in den ersten Reaktor dosiert wird.

In einer siebenundsechzigsten Ausführung betrifft die Erfindung ein Verfahren gemäß einer der ersten bis sechsundsechzigsten Ausführungsform, wobei das Alkylenoxid kontinuierlich oder stufenweise bevorzugt kontinuierlich aus dem ersten Reaktor entnommen wird.

In einer achtundsechzigsten Ausführung betrifft die Erfindung ein Verfahren gemäß einer der dreiundsechzigsten bis siebenundsechzigsten Ausführungsform, wobei die erste aromatische Verbindung, die zweite aromatische Verbindung und/oder die dritte aromatische Verbindung kontinuierlich oder stufenweise bevorzugt kontinuierlich aus dem ersten Reaktor entnommen wird. In einer neunundsechzigsten Ausführung betrifft die Erfindung ein Verfahren gemäß einer der dreiundsechzigsten bis achtundsechzigsten Ausführungsform, wobei das Alkylenoxid und die halogenierte, bevorzugt das Alkylenoxid und die erste aromatische Verbindung, die zweite aromatische Verbindung und/oder die dritte aromatische Verbindung kontinuierlich oder stufenweise bevorzugt kontinuierlich aus dem ersten Reaktor entnommen wird.

In einer siebzigsten Ausführung betrifft die Erfindung ein Verfahren gemäß einer der ersten bis neunundsechzigsten Ausführungsform, wobei der Katalysator (A) kontinuierlich oder stufenweise bevorzugt kontinuierlich in den Reaktor dosiert wird.

In einer einundsiebzigsten Ausführung betrifft die Erfindung ein Verfahren gemäß einer der ersten bis siebzigsten Ausführungsform, wobei der erste Reaktor ein Rührkessel, Strömungsrohr, Blasensäule, Schlaufenreaktor, Rieselbett-Reaktor, Sprühturm-Reaktor oder Fallfilm-Reaktor, ist. In einer zweiundsiebzigsten Ausführung betrifft die Erfindung ein Verfahren gemäß einer der ersten bis einundsiebzigsten Ausführungsform, wobei das Arenoxid, das Pyridinoxid und/oder das Pyrimidin-N-oxid in einem zweiten Reaktor hergestellt wird, wobei der zweite Reaktor ungleich dem ersten Reaktor ist.

In einer dreiundsiebzigsten Ausführung betrifft die Erfindung ein Verfahren gemäß zweiundsiebzigsten Ausfürhungsform, wobei die Herstellung des Arenoxids, des Pyridin-N-oxids und/oder das Pyrimidin-N-oxids im zweiten Reaktor bei einer Temperatur von 20 °C bis 250 °C bevorzugt von 50 °C bis 220 °C und besonders bevorzugt von 100 °C bis 200 °C erfolgt.

In einer vierundsiebzigsten Ausführung betrifft die Erfindung ein Verfahren gemäß der zweiundsiebzigsten oder dreiundsiebzigsten Ausführungsform, wobei die Herstellung des Arenoxids, des Pyridin-N-oxids und/oder das Pyrimidin-N-oxids im zweiten Reaktor bei einem Druck von 1 bara bis 200 bara bevorzugt von 1 bara bis 100 bara und besonders bevorzugt von 1 bara bis 60 bara erfolgt.

In einer fünfundsiebzigsten Ausführung betrifft die Erfindung ein Verfahren gemäß einer der zweiundsiebzigsten bis vierundsiebzigsten Ausführungsform, wobei die Herstellung des Arenoxids, des Pyridin-N-oxids und/oder das Pyrimidin-N-oxids im zweiten Reaktor in einem Zeitraum von 6 min bis 48 h, bevorzugt von 6 min bis 24 h und besonders bevorzugt von 6 min bis 3 h erfolgt.

In einer sechsundsiebzigsten Ausführung betrifft die Erfindung ein Verfahren gemäß einer der zweiundsiebzigsten bis fünfundsiebzigsten Ausführungsform, wobei die erste aromatische Verbindung, die zweite aromatische Verbindung und/oder die dritte aromatische Verbindung kontinuierlich oder stufenweise bevorzugt kontinuierlich in den zweiten Reaktor dosiert wird.

In einer siebenundsiebzigsten Ausführung betrifft die Erfindung ein Verfahren gemäß einer der zweiundsiebzigsten bis sechsundsiebzigsten Ausführungsform, wobei Sauerstoff oder das Sauerstoff-haltige Gasgemisch kontinuierlich oder stufenweise bevorzugt kontinuierlich in den zweiten Reaktor dosiert wird.

In einer achtundsiebzigsten Ausführung betrifft die Erfindung ein Verfahren gemäß einer der zweiundsiebzigsten bis siebenundsiebzigsten Ausführungsform, wobei die erste aromatische Verbindung, die zweite aromatische Verbindung und/oder die dritte aromatische Verbindung und Sauerstoff oder das Sauerstoff-haltige Gasgemisch kontinuierlich oder stufenweise bevorzugt kontinuierlich in den zweiten Reaktor dosiert wird.

In einer neunundsiebzigsten Ausführung betrifft die Erfindung ein Verfahren gemäß einer der zweiundsiebzigsten bis achtundsiebzigsten Ausführungsform, wobei das Arenoxid, das Pyridin-N-oxid und/oder das Pyrimidin-N-oxid kontinuierlich oder stufenweise bevorzugt kontinuierlich aus dem zweiten Reaktor entnommen wird.

In einer achtzigsten Ausführung betrifft die Erfindung ein Verfahren gemäß einer der zweiundsiebzigsten bis neunundsiebzigsten Ausführungsform, wobei der Katalysator (C) kontinuierlich oder stufenweise bevorzugt kontinuierlich in den zweiten Reaktor dosiert wird.

In einer einundachtzigsten Ausführung betrifft die Erfindung ein Verfahren gemäß einer der zweiundsiebzigsten bis achtzigsten Ausführungsform, wobei der zweite Reaktor ein Rührkessel, Strömungsrohr, Blasensäule, Schlaufenreaktor, Rieselbett-Reaktor, Sprühturm-Reaktor oder Fallfilm-Reaktor, ist.

In einer zweiundachtzigsten Ausführung betrifft die Erfindung ein Verfahren gemäß einer der zweiundsiebzigsten bis einundachtzigsten Ausführungsform, wobei das im zweiten Reaktor hergestellte Arenoxid, Pyridin-N-oxid und/oder Pyrimidin-N-oxid kontinuierlich oder stufenweise bevorzugt kontinuierlich in ersten Reaktor dosiert wird.

In einer dreiundachtzigsten Ausführung betrifft die Erfindung ein Verfahren gemäß einer der ersten bis zweiundachtzigsten Ausführungsform, wobei das Alken eine oder mehrere Verbindung(en) ist und ausgewählt wird aus der Gruppe bestehend aus Ethen, Propen, Buten, 1-Okten, Butadien, 1,4-Butandioldiallylether, Allylchlorid, Allylalcohol, Styrol, Cyclopenten, Cyclohexen, Phenyl-allylether, Diallylether, n-Butyl-allylether, tert-Butyl-allylether, Bisphenol-A diallyl ether, Resorcindiallylether, Triphenylolmethan-triallylether, Cyclohexan-1,2-dicarbonsäure-bis-(allylester), Isocyanursäuretris-(prop-2,3-en)-ester und Gemische dieser Alkene bevorzugt Ethen, Propen und Allylchlorid und besonders bevorzugt Propen.

### Beispiele

### Verwendete Chemikalien

Kupfer, Pulver, 99,999% Sigma Aldrich
Kupfermonochlorid, ≥ 99,995%, Sigma Aldrich
Silber, Pulver, 2-3,5 µm, ≥ 99,9%, Sigma Aldrich
Eisen(III)-chlorid, ≥ 99,99%, Sigma Aldrich
Chrom(III)-chlorid, 99,99%, Sigma Aldrich
Ruthenium(III) chlorid hydrat, 99.98%, Sigma Aldrich

Alle Chemikalien wurden so eingesetzt wie sie erhalten wurden.

### Gaschromatographie-Analytik

Die gaschromatographische Analyse (kurz GC) von Flüssig- und Gasproben wurde in Anlehnung an "Determine Impurities in High-Purity Propylene Oxide with Agilent J&W PoraBOND U" von Dianli Ma, Ningbo ZRCC Lyondell Chemical Co., Ltd Zhejiang, China, und Yun Zou, Hua Wu, Agilent Technologies, Inc. durchgeführt. Auf Basis von GC wurden Umsätze an Propene, Ausbeuten an Propylenoxide und Selektivitäten ermittelt.

### Simulationsmethode

Alle quantenmechanischen Berechnungen wurden dabei mit dem Programmpaket TURBOMOLE Version 7.4.1 der Firma Cosmologic GmbH & Co. KG durchgeführt. Die verwendete Dichtefunktionaltheoretische Methode war das TPSS-Dichtefunktional, ausgeführt als unrestricted-DFT für Spincontamination offenschaliger Systeme, mit einem def2-SVP-Basissatz, so wie standardmäßig im Turbomole-Programmpaket implementiert. Die erhaltenen Energien wurden mit der beschrieben DFT-Methode und einem Basissatz der Qualität def2-TZVP verfeinert.

Abb. 1: Input-Geometrie für die quantenchemischen Berechnungen der Übergangszustände des katalysierten Sauerstoffübertrags.

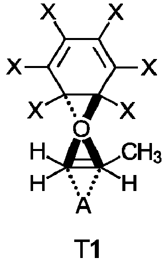

Übergangszustände wurden mittels Gradient basiertem Monte Carlo berechnet, wie in Anmeldung WO 2020/079094 A2 beschrieben. Dazu wurde eine Struktur gemäß des Übergangszustand T1 gezeichnet (Abbildung 1). Die fett eingezeichneten Bindungen wurden auf einen Atomabstand von 1,90 Å (190 pm) gesetzt und die so erhaltene Struktur in kartesische Koordinaten übersetzt. Die Atomindices im kartesischen Koordinatensatz der in Abbildung 1 fett eingezeichneten Bindungen wurden in dem Gradient basierten Monte Carlo Programm als Funktionsraum gesetzt und die Monte Carlo Prozedur so lange ausgeführt bis die entsprechenden Übergangszustände T1 erhalten wurden. Danach wurden die so erhaltenen Kartesischen Koordinaten der Strukturen T1 manipuliert um die dazugehörigen Edukt-katalysatorkomplexe bzw. die dazugehörigen Katalysator-Produktkomplexe zu erhalten. Dazu wurde die fett gezeichnete Bindung (Abbildung 1) zwischen Sauerstoff und dem Aromatischen Kohlenstoffatom des Halogenaromaten um 0,20 Å (20 pm) i) verlängert und ii) verkürzt. Die so erhaltenen Strukturen i) und ii) wurden in Form kartesischer Koordinaten gebracht, einer Geometrieoptimierung mit der beschriebenen DFT-Methode unterworfen und die erhaltenen Geometrien für die Berechnung der Aktivierungsenergien verwendet.

Als Oxidationsvermittler für die selektive Oxidation Propen zu Propylenoxid wurden mittels quantenchemischer Simulationen (Ausführungsbeispiele) Arenoxide von Hexafluorbenzol und Hexachlorbenzol sowie Pentafluorpyridin-N-oxid als geeignet identifiziert. Hexafluorbenzol und Hexachlorbenzol lassen sich gut in die jeweiligen Arenoxide überführen, sind gegen ungewollte Zerfallsreaktionen reaktionsträge und übertragen den atom. Sauerstoff selektiv an das Propen unter ausschließlicher PO-Bildung (vgl. Abb. 2). Analoges gilt für Pentafluorpyridin-N-oxid. Arenoxide verschiedener halogenierter Benzole sind auch kommerziell erhältlich.
Zur experimentellen Beweisführung einer Nutzbarkeit derartiger Arenoxide und strukturell verwandter Pyridin-N-oxiden zur Bildung von Alkylenoxid wurden quantenchemische Simulationen durchgeführt bei denen der Übertrag des gebundenen Sauerstoffs auf das Propen untersucht wurde. Im Fall der Arenoxide erfolgt dabei ein Übertrag des gebundenen Sauerstoffatoms auf die Doppelbindung des Propens wobei eine Rearomatisierung des Aromatischen Kohlenwasserstoffs stattfindet. Im Fall von Pentafluorpyridin-N-oxid erfolgt eine Bildung des elektronisch ungeladenen Pyridinbindungssystems. Dabei dient die Rückbildung der aromatischen Systeme als Triebkraft zur Übertragung des Sauerstoffs auf das Propen. In beiden Verbindungsklassen erfolgt eine umfassende und selektive Rückgewinnung der Ausgangskomponenten durch die Sauerstoffübertragung. Eine Rückgewinnung und erneute Oxidation unter Bildung der Oxidationsvermittlerstruktur ist daher immer möglich. Die Ausgangsverbindungen der Oxidationsvermittler werden recycelt.

Abb.2: Reaktionssequenz der katalytischen Bildung des Arenoxids in Gegenwart des Katalysators (D) gefolgt von der Oxidation von Propen mit einem Arenoxid mittels unter Einsatz des Katalysators (A).

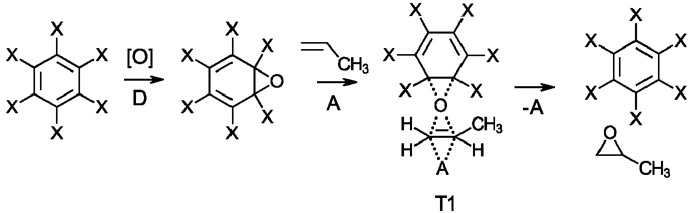

**Tab.1: Übersicht über simulativ berechnete Aktivierungsenergien des Sauerstoffübertrags auf Propen mit verschiedenen Oxidationsvermittlern.**

| Oxidationsvermittler-Oxid | **Ea, simuliert** |
|---|---|
| | [kcal/mol] |
| Hexafluorbenzoloxid | 27.3 |
| Hexachlorbenzoloxid | 21.6 |
| Pentafluorpyridin-N-Oxid | 32.5 |

Die in Tabelle 1 gezeigten Werte legen nahe dass im Fall von Hexachlorbenzoloxid bereits ohne weitere Hilfsmittel wie bestimmte Katalysatoren eine Alkoxylierung von Propen (Epoxidierung von Propen, Propoxylierung) zu allein durch entsprechende Reaktionstemperaturen erreichen ist. Für Hexafluorbenzoloxid und Pentafluorpyridin-N-oxid hingegen indizieren die berechneten Aktivierungsenergien die zusätzliche Verwendung eines geeigneten Katalysators um die Epoxidierung von Propen zu ermöglichen.
Als Katalysator (A) geeignet wurden mittels quantenchemischer Simulationen (Ausführungsbeispiele) metallisches Silber, metallisches Kupfer, Kupfer-(I)-Clorid, Eisen-(III)-Chlorid und Chrom-(III)-Chlorid identifiziert.

**Tabelle 2: Übersicht über simulativ berechnete Aktivierungsenergien des Sauerstoffübertrags von Hexafluorbenzoloxid auf Propen mit verschiedenen Katalysatoren (A).**

| **Katalysator (A)** | **Ea,simuliert** |
|---|---|
| | [kcal/mol] |
| Unkatalysiert, Hexafluorbenzoloxid | 27.3 |
| Ag(0) | 17.4 |
| Cu(0) | 18.2 |
| NiCl₂En₂ (Vgl.) | 28.0 |
| Fe(III)Cl₃ | 14.3 |
| Cr(III)Cl₃ | 17.3 |

Die berechneten Werte in Tabelle2 zeigen dass die Aktivierungsenergie der unkatalysierten Reaktion von 27.3 kcal/mol durch die eingesetzten Katalysatoren (A) deutlich verringert werden kann. Außerdem verdeutlichen die simulierten Ergebnisse dass die Aktivierungsenergien deutlich von der jeweiligen Auswahl des Katalysators (A) abhängen. Im Falle des Hexachlorbezoloxids ist die berechnete Aktivierungsenergie von 21.6 Kcal/mol bereits ohne Einsatz eines Katalysator (A) vorteilhaft.

**Tabelle 3: Übersicht über simulativ berechnete Aktivierungsenergien des Sauerstoffübertrags von Pentafluorpyridin-N-oxid auf Propen.**

| **Katalysator (A)** | **Ea, simuliert(Propoxylierung)** | **Ea, simuliert, Nebenreaktion** |
|---|---|---|
| | [kcal/mol] | [kcal/mol] |
| Unkatalysiert (Vgl.) | 32.5 | |
| Ag(0) | 15.2 | 22.7 |
| Au(0) | 11.2 | 20.2 |
| Cu(0) | 10.3 | 19.9 |
| Cu(II)Cl₂ | 14.0 | 33.7 |
| Cr(III)Cl₃ | 15.9 | 19.6 |
| Co(0) (Vgl.) | 26.3 | 30.1 |
| Co(II)Cl₂ | 20.5 | 30.3 |
| Ru(III)Cl₃ (Vgl.) | _¹⁾ | 25.3 |
| Ru(II)Cl₂ (Vgl.) | _¹⁾ | - |
| [Pt(II)Cl₃]⁻ (Vgl.) | 41.6 | - |

| | | |
|---|---|---|
| 1) Führt an Stelle von Propylenoxid oder entsprechender Intermediate weiter zu C-C-Spaltung des Propylens (Bindungsabstand auf der Produktseite beträgt in den Simulationen 3.112Å bis 4.124 Å, während der berechnete C-C-Bindungsabstand bei dem Propylenoxid 1.475Å beträgt). | | |

Tabelle 3 zeigt die simulierten Aktivierungsenergien für die Epoxidierung von Propen mit Hexafluopyridin-N-oxid mit und ohne Katalysatoren. Die berechneten Werte zeigen dass die Aktivierungsenergie der unkatalysierten Reaktion von 32.5 kcal/mol durch die eingesetzten Katalysatoren A zum Teil deutlich verringert werden kann (vgl. Spalte "Ea, simuliert (Propoxylierung)").

### 1) Alkoxylierung von Propen mit Hexachlorbenzoloxid:

Hexachlorbenzoloxid wurde nach dem Stand der Technik durch katalytische Oxidation von Hexachlorbenzol gewonnen. Es wurde eine 1 M Lösung von Hexachlorbenzoloxid in Perfluorodecalin erzeugt. Nun wurde in einem druckfesten 1L-Reaktor mit Rührwerk, Überdruckabsicherung, Druckaufnehmer, Steigrohr zur Flüssigkeitsentnahme sowie Be- und Entgasungsleitungen 200 mL Perfluorodecalin unter Inertbedingungen vorgelegt. Das Reaktionsgefäß wurde anschließend mit 0,400 mol Propen beaufschlagt (ca. 11 bar). Die Reaktorinnentemperatur wurde auf 135°C eingeregelt und es wurden über 30 min 0,400 mol Hexachlorbenzoloxid in Form der zuvor präparierten Perfluorodecalin-Lösung hinzugegeben. Der Reaktionsfortschritt und -Endpunkt wurde anhand des Druckverlaufs und durch Entnahme von Flüssig- und Gasproben und deren Analyse per GC analysiert ermittelt. Nach Beendigung der Reaktion wurde der Reaktor auf 40 °C abgekühlt, anschließend entspannt und das Reaktionsprodukt Propylenoxid über die Entgasungsleitung in eine gekühlte Vorlage abdestilliert. Die theoretische Ausbeute an Propylenoxid beträgt dabei 23,2 g.

### 2) Alkoxylierung von Propen mit Hexachlorbenzoloxid:

In einem druckfesten 1L-Reaktor mit Rührwerk, Überdruckabsicherung, Druckaufnehmer, Steigrohr zur Flüssigkeitsentnahme sowie Be- und Entgasungsleitungen 200 mL Perfluorodecalin, 0,400 mol Hexachlorbenzol, 0,040 mol Silber (Pulver, 2-3,5 µm) unter Inertbedingungen vorgelegt und kräftig gerührt. Das Reaktionsgefäß wurde anschließend mit 0,400 mol Sauerstoff beaufschlagt. Die Reaktorinnentemperatur wurde auf 200°C eingeregelt und der Druckverlauf bis zur Konstanz beobachtet. Anschließend wurde auf 135 °C abgekühlt, mit Stickstoff inertisiert und 0,400 mol Propen zugegeben. Der Reaktionsfortschritt und -Endpunkt wurde anhand des Druckverlaufs und durch Entnahme von Flüssig- und Gasproben und deren Analyse per GC analysiert ermittelt. Nach Beendigung der Reaktion wurde der Reaktor auf 40 °C abgekühlt, anschließend entspannt und das Reaktionsprodukt Propylenoxid über die Entgasungsleitung in eine gekühlte Vorlage abdestilliert. Die theoretische Ausbeute an Propylenoxid beträgt dabei 23,2 g.

### 3) Alkoxylierung von Propen mit Hexafluorbenzoloxid:

Hexafluorbenzoloxid wurde durch partielle Oxidation von Hexafluorbenzol mit Sauerstoff in einem Druckreaktor gewonnen. Der Umsatz an Hexafluorbenzol wurde dabei so gewählt das die Konzentration an Hexafluorbenzoloxid 1 mol/L (1 M) betrug. Nun wurde in einem druckfesten 1L-Reaktor mit Rührwerk, Überdruckabsicherung, Druckaufnehmer, Steigrohr zur Flüssigkeitsentnahme sowie Be- und Entgasungsleitungen 200 mL Hexafluorbenzol und je Versuch 0,020 mol des jeweiligen, in Tabelle 2 als Ausführungsbsp. gekennzeichneten, Katalysatoren unter Inertbedingungen vorgelegt. Das Reaktionsgefäß wurde anschließend mit 0,400 mol Propen beaufschlagt (ca. 11 bar). Die Reaktorinnentemperatur wurde auf 60°C eingeregelt und es wurden über 30 min 0,400 mol Hexafluorbenzoloxid in Form der zuvor präparierten Lösung hinzugegeben. Der Reaktionsfortschritt und -Endpunkt wurde anhand des Druckverlaufs und durch Entnahme von Flüssig- und Gasproben und deren Analyse per GC analysiert ermittelt. Nach Beendigung der Reaktion wurde der Reaktor auf 40 °C abgekühlt, anschließend entspannt und das Reaktionsprodukt Propylenoxid über die Entgasungsleitung in eine gekühlte Vorlage abdestilliert. Die theoretische Ausbeute an Propylenoxid beträgt in allen fünf Versuchen 23,2 g.

### 4) Alkoxylierung von Propen mit Hexafluorbenzoloxid:

Die kontinuierliche Epoxidierung von Propen mit Hexafluorbenzoloxid wurde analog zu Versuchsvorschrift 3 durchgeführt. Jedoch wurden das Hexafluorbenzoloxid und das Propen kontinuierlich in den Reaktor dosiert (Stoffmengenverhältnis 1,05:1) und das Reaktionsgemisch volumetrisch in gleicher Weise entnommen und destillativ aufgearbeitet. Die Stoff- und Volumenströme wurden dabei so eingeregelt, dass ein Verweilzeit von 90 min resultierte. Nach 6 Stunden befand die Reaktion im stationären Zustand. Umsätze und Selektivitäten analog zu Versuch 3 wurden in allen Versuchen mit den unterschiedlichen erfindungsgemäßen Katalysatoren aus Tabelle 2 beobachtet.

### 4) Alkoxylierung von Propen mit Hexafluorbenzoloxid:

Die kontinuierliche Epoxidierung von gasförmigen Propen mit gasförmigen Hexafluorbenzoloxid wurde analog zu Versuchsvorschrift 4 durchgeführt. Jedoch wurden das Hexafluorbenzoloxid und das Propen verdampft und kontinuierlich in einen inertisierten, druckfesten und druckabgesicherten Strömungsreaktor eingebracht, der Silber (Pulver, 2-3,5 µm) als festem Katalysator enthielt. Das Stoffmengenverhältnis betrug dabei 5:1 Hexafluorbenzoloxid zu Propen und eine Reaktionstemperatur von 120 °C wurde eingeregelt. Teilumsätze an Propen und Propylenoxid-Selektivitäten analog zu Versuch 3 und 4 wurden beobachtet. Eine genaue Bestimmung von Verweilzeiten fand nicht statt.

### 5) Epoxidierung von Propen mit Pentafluorpyidin-N-oxid:

Pentafluorpyidin-N-oxid wurde analog wie im Stand der Technik für N-Oxide beschrieben (Chem. Commun. 2002, 1040-1041) dargestellt und als 1 M Dichlorethan-Lösung bereitgestellt. Nun wurde in einem druckfesten 1L-Reaktor mit Rührwerk, Überdruckabsicherung, Druckaufnehmer, Steigrohr zur Flüssigkeitsentnahme sowie Be- und Entgasungsleitungen 200 mL Dichlorethan und je Versuch 0,020 mol des jeweiligen, in Tabelle 3 als Ausführungsbsp. gekennzeichneten, Katalysatoren unter Inertbedingungen vorgelegt. Das Reaktionsgefäß wurde anschließend mit 0,400 mol Propen beaufschlagt (ca. 11 bar). Die Reaktorinnentemperatur wurde auf 60°C eingeregelt und es wurden über 30 min 0,400 mol Pentafluorpyidin-N-oxid in Form der zuvor präparierten Lösung hinzugegeben. Der Reaktionsfortschritt und -Endpunkt wurde anhand des Druckverlaufs und durch Entnahme von Flüssig- und Gasproben und deren Analyse per GC analysiert ermittelt. Nach Beendigung der Reaktion wurde der Reaktor auf 40 °C abgekühlt, anschließend entspannt und das Reaktionsprodukt Propylenoxid über die Entgasungsleitung in eine gekühlte Vorlage abdestilliert. Die theoretische Ausbeute an Propylenoxid beträgt in allen fünf Versuchen 23,2 g.

## Patentansprüche

1. Verfahren zur Herstellung eines Alkylenoxids durch Umsetzung eines Alkens mit einem Arenoxid, Pyridin-N-oxid und/oder Pyrimidin-N-oxid bevorzugt mit einem Arenoxid und/oder Pyridin-N-oxid bevorzugt in Gegenwart eines Katalysators (A) in einem ersten Reaktor, wobei der Katalysator (A) ein Metall (A-1) und/oder ein Metallsalz (A-2) umfasst, wobei das Metall (A-1) Kupfer, Silber und/oder Gold ist,
wobei das Metallsalz (A-2) Chrom (Cr), Eisen (Fe), Cobalt (Co) und/oder Kupfer (Cu), Kation(en) umfasst,
und wobei die Umsetzung in Abwesenheit von Sauerstoff oder einem Sauerstoff-haltigen Gasgemisch erfolgt.

2. Verfahren gemäß Anspruch 1, wobei das Alkylenoxid eine oder mehrere Verbindung(en) ist und ausgewählt wird aus der Gruppe bestehend aus Ethylenoxid, Propylenoxid, 1,2-Butylenoxid, 1,2-Pentylenoxid, 1,2-Hexylenoxid, 1,2-Heptylenoxid und 1,2-Octylenoxid bevorzugt Ethylenoxid und Propylenoxid besonders bevorzugt Propylenoxid.

3. Verfahren gemäß Anspruch 1 oder 2, wobei das Alken eine oder mehrere Verbindung(en) ist und ausgewählt wird aus der Gruppe bestehend aus Ethen, Propen, Buten, 1-Okten, Butadien, 1,4-Butandioldiallylether, Allylchlorid, Allylalcohol, Styrol, Cyclopenten, Cyclohexen, Phenyl-allylether, Diallylether, n-Butyl-allylether, tert-Butyl-allylether, Bisphenol-A diallyl ether, Resorcindiallylether, Triphenylolmethan-triallylether, Cyclohexan-1,2-dicarbonsäure-bis-(allylester), Isocyanursäuretris-(prop-2,3-en)-ester und Gemische dieser Alkene bevorzugt Ethen, Propen und Allylchlorid und besonders bevorzugt Propen.

4. Verfahren gemäß einem der Ansprüche 1 bis 3, wobei das Arenoxid eine oder mehrere Verbindung(en) ist und ausgewählt wird aus der Gruppe bestehend aus Hexafluorbenzoloxid, Hexachlorbenzoloxid, 1-Brom-2,3,4-trifluorbenzoloxid, Pentafluorbenzoloxid, 1,3,5-Trichlor-2,4,6-trifluorbenzoloxid, 1,3,5-Trfluorbenzoloxid, 1,2-Dibrom-3,5-difluorbenzoloxid, 1,2,4,5-Tetrafluorbenzoloxid, Brompentafluorbenzoloxid, 1,3,5-Trichlorbenzoloxid, 1-Brom-3,5-dichlrobenzoloxid, Orthodichlorbenzoloxid, 1,2,4,5-Tetrachlorbenzoloxid, 1,2,3-Trichlorbenzoloxid und 1,5-Dichlro-2-fluorbenzoloxid, bevorzugt Hexafluorbenzoloxid, und Hexachlorbenzoloxid.

5. Verfahren gemäß einem der Ansprüche 1 bis 4, wobei das Pyridin-N-oxid eine oder mehrere Verbindung(en) ist und ausgewählt wird aus der Gruppe bestehend aus Pentafluorpyridin-N-Oxid, 2-Brom-3,5-dichlorpyridin-N-oxid, 3-Chlorpyridin-N-oxid, 3,6-Dichlorpyridin-N-oxid, 3,5-Dichlorpyridin-N-oxid, 3-Chlor-2,5,6-trifluorpyridin-N-oxid, 3-Chlo-2,4,5,6-tetrafluorpyridin-1-N-oxid, 3-Chlo-2,4,5,6-tetrafluorpyridin-3-N-oxid, bevorzugt Pentafluorpyridin-N-Oxid.

6. Verfahren gemäß einem der Ansprüche 1 bis 5, wobei das Pyrimidin-N-oxid eine oder mehrere Verbindungen und ausgewählt wird aus der Gruppe bestehend aus 2-Chlorpyrimidin-N-oxid, 2,4-Dichlor-6-methylpyrimidin-1-N-oxid, 2,4-Dichlor-6-methylpyrimidin-3-N-oxid, 2,5-Dichlorpyrimidin-1-N-oxid, 2,5-Dichlorpyrimidin-2-N-oxid.

7. Verfahren gemäß einem der Ansprüche 1 bis 6, wobei der Metall (A-1) Kupfer (Cu), Silber (Ag) und/oder Gold (Au) bevorzugt Silber (Ag) ist.

8. Verfahren gemäß einem der Ansprüche 1 bis 7, wobei das Metallkation des Metallsalzes (A-2) eine Oxidationsstufe von (+I), (+II); (+III) oder (+IV) bevorzugt von (+II); (+III) oder (+IV) aufweist.

9. Verfahren gemäß einem der Ansprüche 1 bis 8, wobei das Metallsalz (A-2) eine oder mehrere Verbindung(en) ist und ausgewählt wird aus der Gruppe bestehend aus Cr₂(SO₄)₃, KCr(SO₄)₂, Cr(NO₃)₃, CrF₃, CrCl₃, FeCl₃, FeBr₃, Eisentriflat, FePO₄, Fe₂(SO4)₃, Fe(NO₃)₃, FeF₃, Eisen-paratoluolsulfonat, CoCl₂, CoBr₂, Co(NO₃)₂, CoBr₂, CoSO₄, CoF₂, Co(BF₄)₂, Co₃(PO₄)₂, CuCl₂, CuSO₄, (CF₃SO₃)₂Cu, CuF₂, Cu(NO₃)₂, Kupfer(II)-pyrophosphat, CuCl, CuI und CuBr bevorzugt CrCl₃, FeCl₃, CoCl₂ und CuCl₂.

10. Verfahren gemäß einem der Ansprüche 1 bis 9, wobei die Herstellung des Alkylenoxids in Gegenwart eines Lösungsmittels erfolgt.

11. Verfahren gemäß einem der Ansprüche 1 bis 9, wobei die Herstellung in Abwesenheit eines Lösungsmittels erfolgt.

12. Verfahren gemäß einem der Ansprüche 1 bis 11, wobei das Alken und das Arenoxid, Pyridin-N-oxid und/oder das Pyrimidin-N-oxid kontinuierlich oder stufenweise bevorzugt kontinuierlich in den ersten Reaktor dosiert wird.

13. Verfahren gemäß einem der Ansprüche 1 bis 12, wobei das Alkylenoxid kontinuierlich oder stufenweise bevorzugt kontinuierlich aus dem ersten Reaktor entnommen wird.

14. Verfahren gemäß einem der Ansprüche 1 bis 13, wobei das Arenoxid, das Pyridinoxid und/oder das Pyrimidin-N-oxid in einem zweiten Reaktor hergestellt wird, wobei der zweite Reaktor ungleich dem ersten Reaktor ist.

15. Verfahren gemäß Anspruch 14, wobei das im zweiten Reaktor hergestellte Arenoxid, Pyridin-N-oxid und/oder Pyrimidin-N-oxid kontinuierlich oder stufenweise bevorzugt kontinuierlich in ersten Reaktor dosiert wird.
